# EUROPEAN PATENT SPECIFICATION

(11) **EP 2 854 541 B1**
(45) Date of publication and mention of the grant of the patent: **15.06.2016**
(21) Application number: 13727540.0
(22) Date of filing: 22.05.2013
(51) Int. Cl.: A01N 43/90, C07D 405/12, C07D 209/70, A01P 21/00

(54) **PLANT GROWTH REGULATING COMPOUNDS**
PFLANZENWACHSTUMSREGULIERENDE VERBINDUNGEN
COMPOSÉS RÉGULATEURS DE LA CROISSANCE DES PLANTES

(30) Priority: 24.05.2012 GB 201209307; 16.10.2012 EP 12188735
(43) Date of publication of application: 08.04.2015
(73) Proprietor: Syngenta Participations AG, 4058 Basel (CH)
(72) Inventor: VILLEDIEU-PERCHERON, Emmanuelle, CH-Stein 4332 (CH); LACHIA, Mathilde, Denise, CH-4332 Stein (CH); DE MESMAEKER, Alain, CH-4332 Stein (CH); WOLF, Hanno, Christian, CH-4332 Stein (CH); JUNG, Pierre, Joseph, Marcel, CH-4332 Stein (CH); LANFERMEIJER, Franciscus, 1601 BK Enkhuizen (NL); VAN DEN WIJNGAARD, Paul, NL-1601 BK Enkhuizen (NL); SCREPANTI, Claudio, CH-Stein 4332 (CH)
(74) Representative: Syngenta International AG
(86) International application number: PCT/EP2013/060470
(87) International publication number: WO 2013/174846

(56) References cited:
- WO-A2-2010/125065

## Description

The present invention relates to novel strigolactam derivatives, to processes and intermediates for preparing them, to plant growth regulator compositions comprising them and to methods of using them for controlling the growth of plants and/or promoting the germination of seeds.

Strigolactone derivatives are phytohormones with plant growth regulation and seed germination properties; they have been described, for example, in WO2009/138655, WO2010/125065, WO05/077177, WO06/098626, WO11/125714 and Annual Review of Phytopathology (2010), 48 p.93-117. Strigolactone derivatives, like the synthetic analogue GR24, are known to have effect on the germination of parasitic weeds, such as *Orobanche* species. It is well established in the art that testing for germination of *Orobanche* seeds is a useful test to identify strigolactone analogues (for example, see Plant and Cell Physiology (2010), 51(7) p.1095; and Organic & Biomolecular Chemistry (2009), 7(17), p.3413).

It has now surprisingly been found that certain strigolactam derivatives have properties analogous to strigolactone.

According to the present invention, there is provided a compound of Formula (I) 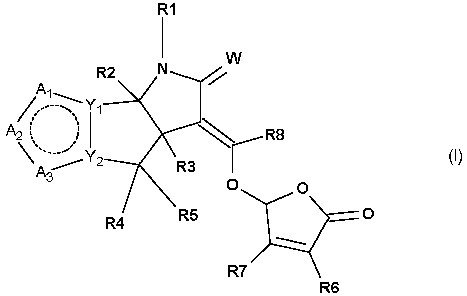 wherein
W is O or S;
R2 and R3 are independently hydrogen, or C₁-C₃ alkyl;
R4 and R5 are independently hydrogen, halogen, nitro, cyano, C₁-C₃ alkyl, C₁-C₃ haloalkyl, C₁-C₃ alkoxy, C₁-C₃ haloalkoxy, hydroxyl, -OC(O)R9, amine, N- C₁-C₃ alkyl amine, or N,N-di-C₁-C₃ alkyl amine;
R9 is hydrogen, C₁-C₆ alkyl, C₁-C₆ alkoxy, or C₁-C₆ haloalkyl;
R6 and R7 are independently hydrogen, C₁-C₃ alkyl, hydroxyl, halogen, or C₁-C₃ alkoxy;
R8 is hydrogen, nitro, cyano, C₁-C₆ alkyl, or C₁-C₆ haloalkyl;
R1 is hydrogen, C₁-C₆ alkoxy, hydroxyl, amine, N- C₁-C₆ alkyl amine, N,N-di- C₁-C₆ alkyl amine, C₁-C₆ alkyl optionally substituted by one to five R10, C₁-C₈ alkylcarbonyl-, or C₁-C₈ alkoxycarbonyl-, aryl optionally substituted by one to five R10, heteroaryl optionally substituted by one to five R10, heterocyclyl optionally substituted by one to five R10, benzyl optionally substituted by one to five R10, C₂-C₈ alkenyl optionally substituted by one to five R10, C₂-C₈ alkynyl optionally substituted by one to five R10, C₃-C₇ cycloalkyl, or C₃-C₁₀ cycloalkyl substituted by one to five R10;
R10 is cyano, nitro, halogen, hydroxyl, C₁-C₆ alkyl, C₁-C₆ alkoxy, C₁-C₆ haloalkoxy, C₁-C₆ haloalkyl, C₁-C₈ alkylthio-, C₁-C₈ haloalkylthio-, C₁-C₈ alkylsulfinyl-, N- C₁-C₆ alkyl amine, N,N-di- C₁-C₆ alkyl amine, C₁-C₈ haloalkylsulfinyl-, C₁-C₈ alkylsulfonyl-, C₁-C₈ haloalkylsulfonyl-, C₁-C₈alkylcarbonyl-, C₁-C₈ alkoxycarbonyl, C₂-C₆ alkenyl or C₂-C₆ alkynyl;
Y₁ and Y₂ are C;
A₁, and A₂ are C-X, wherein each X may be the same or different provided that A₁, A₂, A₃, Y₁ and Y₂ form an aromatic ring;
A₃ is sulphur;
and X is hydrogen, halogen, cyano, nitro, C₁-C₆ haloalkyl, hydroxyl, C₁-C₆ alkoxy, C₁-C₆ haloalkoxy, amine, N- C₁-C₆ alkyl amine, N,N-di- C₁-C₆ alkyl amine, C₁-C₆ alkyl optionally substituted by one to five R10, C₁-C₈ alkylcarbonyl-, or C₁-C₈ alkoxycarbonyl-, N-C₁-C₆ alkyl aminocarbonyl, N,N-di C₁-C₆ alkyl aminocarbonyl, aryl optionally substituted by one to five R10, heteroaryl optionally substituted by one to five R10, heterocyclyl optionally substituted by one to five R10, C₂-C₈ alkenyl optionally substituted by one to five R10, C₂-C₈ alkynyl optionally substituted by one to five R10, C₃-C₇ cycloalkyl, C₃-C₁₀ cycloalkyl substituted by one to five R10, C₁-C₈ alkylthio-, C₁-C₈ haloalkylthio-, C₁-C₈ alkylsulfinyl-, C₁-C₈ haloalkylsulfinyl-, C₁-C₈ alkylsulfonyl-, or C₁-C₈ haloalkylsulfonyl;
or salts or N-oxides thereof.

The compounds of Formula (I) may exist in different geometric or optical isomers (diastereoisomers and enantiomers) or tautomeric forms. This invention covers all such isomers and tautomers and mixtures thereof in all proportions as well as isotopic forms such as deuterated compounds. The invention also covers all salts, N-oxides, and metalloidic complexes of the compounds of Formula (I).

Each alkyl moiety either alone or as part of a larger group (such as alkoxy, alkoxycarbonyl, alkylcarbonyl, alkylaminocarbonyl, dialkylaminocarbonyl, alkylthio, alkylsulfinyl, alkylsulfonyl) is a straight or branched chain and is, for example, methyl, ethyl, *n*-propyl, *n-*butyl, *n*-pentyl, *n*-hexyl, *iso-*propyl, *n*-butyl, *sec*-butyl, *iso*-butyl, *tert*-butyl or *neo*-pentyl. The alkyl groups are preferably C₁ to C₆ alkyl groups, more preferably C₁-C₄ and most preferably C₁-C₃ alkyl groups.

Each alkenyl moiety either alone or as part of a larger group (such as alkoxy, alkoxycarbonyl, alkylcarbonyl, alkylaminocarbonyl, dialkylaminocarbonyl, alkylthio, alkylsulfinyl, alkylsulfonyl) is having at least one carbon-carbon double bond and is, for example, vinyl, allyl. The alkenyl groups are preferably C₂ to C₆ Alkenyl groups, more preferably C₂-C₄ alkenyl groups.

The term "alkenyl", as used herein, unless otherwise indicated, includes alkyl moieties having at least one carbon-carbon double bond wherein alkyl is as defined above

Each alkynyl moiety either alone or as part of a larger group (such as alkoxy, alkoxycarbonyl, alkylcarbonyl, alkylaminocarbonyl, dialkylaminocarbonyl, alkylthio, alkylsulfinyl, alkylsulfonyl) is having at least one carbon-carbon triple bond and is, for example, ethynyl, propargyl. The alkynyl groups are preferably C₂ to C₆ alkynyl groups, more preferably C₂-C₄ alkynyl groups.

The term "alkynyl", as used herein, unless otherwise indicated, includes alkyl moieties having at least one carbon-carbon triple bond wherein alkyl is as defined above.

Halogen is fluorine, chlorine, bromine or iodine.

Haloalkyl groups (either alone or as part of a larger group, such as haloalkoxy or haloalkylthio) are alkyl groups which are substituted with one or more of the same or different halogen atoms and are, for example, -CF₃, -CF₂Cl, -CH₂CF₃ or -CH₂CHF₂.

Hydroxyalkyl groups are alkyl groups which are substituted with one or more hydroxyl group and are, for example, -CH₂OH, -CH₂CH₂OH or -CH(OH)CH₃.

In the context of the present specification the term "aryl" refers to a ring system which may be mono-, bi- or tricyclic. Examples of such rings include phenyl, naphthalenyl, anthracenyl, indenyl or phenanthrenyl. A preferred aryl group is phenyl.

Unless otherwise indicated, alkenyl and alkynyl, on their own or as part of another substituent, may be straight or branched chain and may preferably contain 2 to 6 carbon atoms, preferably 2 to 4, more preferably 2 to 3, and where appropriate, may be in either the (E)- or (*Z*)-configuration. Examples include vinyl, allyl ethynyl and propargyl.

Unless otherwise indicated, cycloalkyl may be mono- or bi-cyclic, may be optionally substituted by one or more C₁-C₆ alkyl groups, and preferably contain 3 to 7 carbon atoms, more preferably 3 to 6 carbon atoms. Examples of cycloalkyl include cyclopropyl, 1-methylcyclopropyl, 2-methylcyclopropyl, cyclobutyl, cyclopentyl and cyclohexyl.

The term "heteroaryl" refers to an aromatic ring system containing at least one heteroatom and consisting either of a single ring or of two or more fused rings. Preferably, single rings will contain up to three and bicyclic systems up to four heteroatoms which will preferably be chosen from nitrogen, oxygen and sulfur. Examples of such groups include pyridyl, pyridazinyl, pyrimidinyl, pyrazinyl, furanyl, thiophenyl, oxazolyl, isoxazolyl, oxadiazolyl, thiazolyl, isothiazolyl, thiadiazolyl, pyrrolyl, pyrazolyl, imidazolyl, triazolyl and tetrazolyl.

The term "heterocyclyl" is defined to include heteroaryl and in addition their unsaturated or partially unsaturated analogues such as 4,5,6,7-tetrahydro-benzothiophenyl, 9H-fluorenyl, 3,4-dihydro-2H-benzo-1,4-dioxepinyl, 2,3-dihydro-benzofuranyl, piperidinyl, 1,3-dioxolanyl, 1,3-dioxanyl, 4,5-dihydro-isoxazolyl, tetrahydrofuranyl and morpholinyl.

Preferred values of W, R2, R3, R4, R5, R6, R7, R8, R1, R10, A₁, A₂, A₃, Y₁, Y₂ and X are, in any combination, as set out below.
W is preferably oxygen.
R2 is preferably hydrogen, methyl, or ethyl; more preferably R2 is hydrogen.
R3 is preferably hydrogen, methyl, or ethyl; more preferably R3 is hydrogen.
R4 is preferably hydrogen, hydroxyl, methyl, or ethyl; more preferably R4 is hydrogen or hydroxyl.
R5 is preferably hydrogen, hydroxyl, methyl, or ethyl; more preferably R5 is hydrogen or hydroxyl.
R6 is preferably hydrogen, methyl, or ethyl; more preferably R6 is methyl.
R7 is preferably hydrogen, methyl, or ethyl; more preferably R7 is hydrogen.
R8 is preferably hydrogen, methyl, or ethyl; more preferably R8 is hydrogen.
R1 is preferably selected from the group consisting of hydrogen, C₁-C₆ alkyl optionally substituted by one to five R10, C₁-C₈ alkylcarbonyl-, or C₁-C₈ alkoxycarbonyl-, aryl optionally substituted by one to five R10, or heteroaryl optionally substituted by one to five R10, heterocyclyl optionally substituted by one to five R10, benzyl optionally substituted by one to five R10. More preferably R1 is hydrogen, C₁-C₆ alkyl optionally substituted by one to five R10, C₁-C₈ alkylcarbonyl-, C₁-C₈ alkoxycarbonyl-, benzyl optionally substituted by one to five R10, or aryl optionally substituted by one to five R10. Even more preferably R1 is hydrogen, methyl, ethyl, phenyl, benzyl, acetate, methoxycarbonyl, or *tert*-butoxycarbonyl. R10 is independently cyano, nitro, halogen, C₁-C₆ alkyl, C₁-C₆ alkoxy, or C₁-C₆ haloalkyl. More preferably R10 is hydrogen, cyano, nitro, chloride, bromine, fluorine, methyl, methoxy or trifluoromethyl.
X is preferably hydrogen, hydroxyl, halogen, cyano, methyl, ethyl, n-propyl, hydroxymethyl, trifluoromethyl or methoxy. More preferably, X is hydrogen, hydroxyl, methyl, trifluoromethyl or methoxy. Most preferably, X is hydrogen, methyl, methoxy or trifluoromethyl.

In a preferred embodiment the compound is of Formula II. 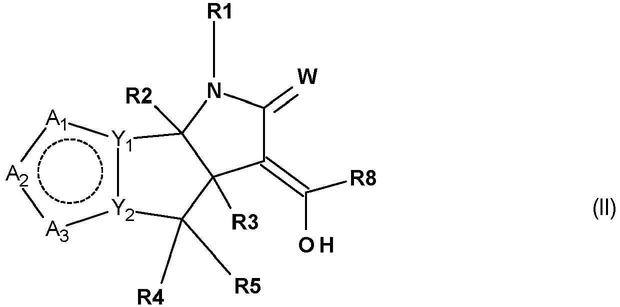 wherein
W is O or S;
R2 and R3 are independently hydrogen, or C₁-C₃ alkyl;
R4 and R5 are independently hydrogen, halogen, nitro, cyano, C₁-C₃ alkyl, C₁-C₃ haloalkyl, C₁-C₃ alkoxy, C₁-C₃ haloalkoxy, hydroxyl, -OC(O)R9, amine, N- C₁-C₃ alkyl amine, or N,N-di-C₁-C₃ alkyl amine;
R9 is hydrogen, C₁-C₆ alkyl, C₁-C₆ alkoxy, or C₁-C₆ haloalkyl;
R8 is hydrogen, nitro, cyano, C₁-C₆ alkyl, or C₁-C₆ haloalkyl;
R1 is hydrogen, C₁-C₆ alkoxy, hydroxyl, amine, N- C₁-C₆ alkyl amine, N,N-di- C₁-C₆ alkyl amine, C₁-C₆ alkyl optionally substituted by one to five R10, C₁-C₈ alkylcarbonyl-, or C₁-C₈ alkoxycarbonyl-, aryl optionally substituted by one to five R10, or heteroaryl optionally substituted by one to five R10, heterocyclyl optionally substituted by one to five R10, benzyl optionally substituted by one to five R10, C₂-C₈ alkenyl optionally substituted by one to five R10, C₂-C₈ alkynyl optionally substituted by one to five R10, C₃-C₇ cycloalkyl, or C₃-C₁₀ cycloalkyl substituted by one to five R10;
R10 is cyano, nitro, halogen, hydroxyl, C₁-C₆ alkyl, C₁-C₆ alkoxy, C₁-C₆ haloalkoxy, C₁-C₆ haloalkyl, C₁-C₈ alkylthio-, C₁-C₈ haloalkylthio-, C₁-C₈ alkylsulfinyl-, N- C₁-C₆ alkyl amine, N,N-di- C₁-C₆ alkyl amine, C₁-C₈ haloalkylsulfinyl-, C₁-C₈ alkylsulfonyl-, C₁-C₈ haloalkylsulfonyl-, C₁-C₈alkylcarbonyl-, C₁-C₈ alkoxycarbonyl, C₂-C₆ alkenyl, or C₂-C₆ alkynyl;
Y₁ and Y₂ are C ;
A₁, and A₂ are C-X, wherein each X may be the same or different, provided that A₁, A₂, A₃, Y₁ and Y₂ form an aromatic ring;
A₃ is sulphur;
and X is hydrogen, halogen, cyano, nitro, C₁-C₆ haloalkyl, hydroxyl, C₁-C₆ alkoxy, C₁-C₆ haloalkoxy, amine, N- C₁-C₆ alkyl amine, N,N-di- C₁-C₆ alkyl amine, C₁-C₆ alkyl optionally substituted by one to five R10, C₁-C₈ alkylcarbonyl-, or C₁-C₈ alkoxycarbonyl-, N-C₁-C₆ alkyl aminocarbonyl, N,N-di C₁-C₆ alkyl aminocarbonyl, aryl optionally substituted by one to five R10, heteroaryl optionally substituted by one to five R10, heterocyclyl optionally substituted by one to five R10, C₂-C₈ alkenyl optionally substituted by one to five R10, C₂-C₈ alkynyl optionally substituted by one to five R10, C₃-C₇ cycloalkyl, C₃-C₁₀ cycloalkyl substituted by one to five R10, C₁-C₈ alkylthio-, C₁-C₈ haloalkylthio-, C₁-C₈ alkylsulfinyl-, C₁-C₈ haloalkylsulfinyl-, C₁-C₈ alkylsulfonyl-, or C₁-C₈ haloalkylsulfonyl;
or salts or N-oxides thereof.

The preferences for A₁, A₂, A₃, Y₁, Y₂, R1, R2, R3, R4, R5, R8, X and W are the same as the preferences set out for the corresponding substituents of the compounds of the Formula (I).
Table 1 below includes examples of compounds of Formula (I) wherein W is O, R2 is H, R3 is H, R4 is H, R5 is H, R6 is methyl, R7 is H, R8 is H and A₁, A₂, A₃, Y₁, Y₂ and R1 are as defined, some of which fall within the scope of the present invention.

**Table 1**

| | | | | | | |
|---|---|---|---|---|---|---|
| 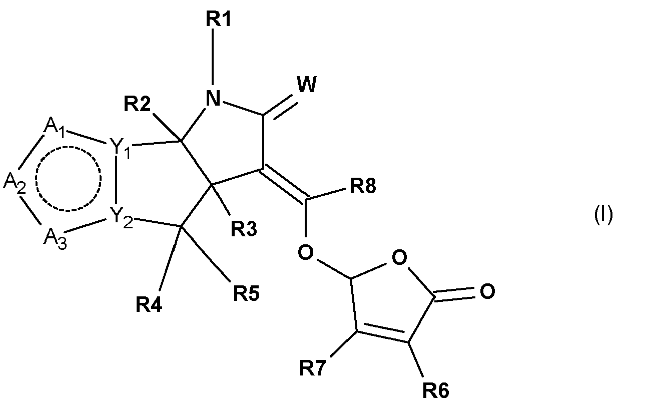 | | | | | | |

| **Compound** | **R1** | **Y₁** | **Y₂** | **A₁** | **A₂** | **A₃** |
|---|---|---|---|---|---|---|
| 1.00 | H | C | C | C-H | C-H | S |
| 1.01 | H | C | C | C-H | C-H | O |
| 1.02 | H | C | C | C-H | C-H | N-H |
| 1.03 | H | C | C | C-H | C-H | N-Me |
| 1.04 | H | C | C | C-H | S | C-H |
| 1.05 | H | C | C | C-H | O | C-H |
| 1.06 | H | C | C | C-H | N-H | C-H |
| 1.07 | H | C | C | C-H | N-Me | C-H |
| 1.08 | H | C | C | S | C-H | C-H |
| 1.09 | H | C | C | O | C-H | C-H |
| 1.10 | H | C | C | N-H | C-H | C-H |
| 1.11 | H | C | C | N-Me | C-H | C-H |
| 1.12 | H | C | C | C-H | N | O |
| 1.13 | H | C | C | C-H | O | N |
| 1.14 | H | C | C | N | O | C-H |
| 1.15 | H | C | C | O | N | C-H |
| 1.16 | H | C | C | C-H | N | S |
| 1.17 | H | C | C | C-H | S | N |
| 1.18 | H | C | C | N | S | C-H |
| 1.19 | H | C | C | S | N | C-H |
| 1.20 | H | C | C | O | C-H | N |
| 1.21 | H | C | C | N | C-H | O |
| 1.22 | H | C | C | S | C-H | N |
| 1.23 | H | C | C | N | C-H | S |
| 1.24 | H | C | C | C-H | N | N-H |
| 1.25 | H | C | C | C-H | N | N-Me |
| 1.26 | H | C | C | N | C-H | N-H |
| 1.27 | H | C | C | N | C-H | N-Me |
| 1.28 | H | C | C | N-H | C-H | N |
| 1.29 | H | C | C | N-Me | C-H | N |
| 1.30 | H | C | C | N | N-H | C-H |
| 1.31 | H | C | C | N | N-Me | C-H |
| 1.32 | H | C | C | N-Me | N | N |
| 1.33 | H | C | C | N-H | N | N |
| 1.34 | H | C | C | N | N | N-Me |
| 1.35 | H | C | C | N | N | N-H |
| 1.36 | H | C | N | N | N | N |
| 2.00 | H | C | C | C-H | C-Me | S |
| 2.01 | H | C | C | C-H | C-Me | O |
| 2.02 | H | C | C | C-Me | C-H | S |
| 2.03 | H | C | C | C-Me | C-H | O |
| 2.04 | H | C | C | C-H | C-Me | N-H |
| 2.05 | H | C | C | C-H | C-Me | N-Me |
| 2.06 | H | C | C | S | C-H | C-Me |
| 2.07 | H | C | C | O | C-H | C-Me |
| 2.08 | H | C | C | N-H | C-H | C-Me |
| 2.09 | H | C | C | N-Me | C-H | C-Me |
| 2.10 | H | C | C | S | C-Me | C-H |
| 2.11 | H | C | C | O | C-Me | C-H |
| 2.12 | H | C | C | N-H | C-Me | C-H |
| 2.13 | H | C | C | N-Me | C-Me | C-H |
| 2.14 | H | C | C | C-Me | N | O |
| 2.15 | H | C | C | C-Me | O | N |
| 2.16 | H | C | C | N | O | C-Me |
| 2.17 | H | C | C | O | N | C-Me |
| 2.18 | H | C | C | C-Me | N | S |
| 2.19 | H | C | C | C-Me | S | N |
| 2.20 | H | C | C | N | S | C-Me |
| 2.21 | H | C | C | S | N | C-Me |
| 2.22 | H | C | C | O | C-Me | N |
| 2.23 | H | C | C | N | C-Me | O |
| 2.24 | H | C | C | S | C-Me | N |
| 2.25 | H | C | C | N | C-Me | S |
| 2.26 | H | C | C | C-Me | N | N-H |
| 2.27 | H | C | C | C-Me | N | N-Me |
| 2.28 | H | C | C | N | C-Me | N-H |
| 2.29 | H | C | C | N | C-Me | N-Me |
| 2.30 | H | C | C | N-H | C-Me | N |
| 2.31 | H | C | C | N-Me | C-Me | N |
| 2.32 | H | C | C | N | N-H | C-Me |
| 2.33 | H | C | C | N | N-Me | C-Me |
| 3.00 | Me | C | C | C-H | C-H | S |
| 3.01 | Me | C | C | C-H | C-H | O |
| 3.02 | Me | C | C | C-H | C-H | NH |
| 3.03 | Me | C | C | C-H | C-H | N-Me |
| 3.04 | Me | C | C | C-H | S | C-H |
| 3.05 | Me | C | C | C-H | O | C-H |
| 3.06 | Me | C | C | C-H | N-H | C-H |
| 3.07 | Me | C | C | C-H | N-Me | C-H |
| 3.08 | Me | C | C | S | C-H | C-H |
| 3.09 | Me | C | C | O | C-H | C-H |
| 3.10 | Me | C | C | N-H | C-H | C-H |
| 3.11 | Me | C | C | N-Me | C-H | C-H |
| 3.12 | Me | C | C | C-H | N | O |
| 3.13 | Me | C | C | C-H | O | N |
| 3.14 | Me | C | C | N | O | C-H |
| 3.15 | Me | C | C | O | N | C-H |
| 3.16 | Me | C | C | C-H | N | S |
| 3.17 | Me | C | C | C-H | S | N |
| 3.18 | Me | C | C | N | S | C-H |
| 3.19 | Me | C | C | S | N | C-H |
| 3.20 | Me | C | C | O | C-H | N |
| 3.21 | Me | C | C | N | C-H | O |
| 3.22 | Me | C | C | S | C-H | N |
| 3.23 | Me | C | C | N | C-H | S |
| 3.24 | Me | C | C | C-H | N | N-H |
| 3.25 | Me | C | C | C-H | N | N-Me |
| 3.26 | Me | C | C | N | C-H | N-H |
| 3.27 | Me | C | C | N | C-H | N-Me |
| 3.28 | Me | C | C | N-H | C-H | N |
| 3.29 | Me | C | C | N-Me | C-H | N |
| 3.30 | Me | C | C | N | N-H | C-H |
| 3.31 | Me | C | C | N | N-Me | C-H |
| 3.33 | Me | C | C | N-Me | N | N |
| 3.34 | Me | C | C | N-H | N | N |
| 3.35 | Me | C | C | N | N | N-Me |
| 3.36 | Me | C | C | N | N | N-H |
| 3.37 | Me | C | N | N | N | N |

Table 2 below includes examples of compounds of Formula (II) wherein W is O, R2 is H, R3 is H, R4 is H, R5 is H, R8 is H and R1, A₁, A₂, A₃, Y₁, and Y₂ are as defined, some of which fall within the scope of the present invention.

**Table 2**

| | | | | | | |
|---|---|---|---|---|---|---|
| 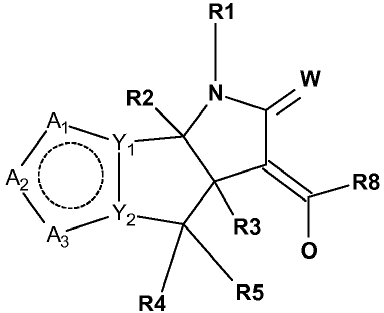 | | | | | | |

| **Compound** | **R1** | **Y₁** | **Y₂** | **A₁** | **A₂** | **A₃** |
|---|---|---|---|---|---|---|
| 1.00 | H | C | c | C-H | C-H | S |
| 1.01 | H | C | C | C-H | C-H | O |
| 1.02 | H | C | C | C-H | C-H | N-H |
| 1.03 | H | C | C | C-H | C-H | N-Me |
| 1.04 | H | C | C | C-H | S | C-H |
| 1.05 | H | C | C | C-H | O | C-H |
| 1.06 | H | C | C | C-H | N-H | C-H |
| 1.07 | H | C | C | C-H | N-Me | C-H |
| 1.08 | H | C | C | S | C-H | C-H |
| 1.09 | H | C | C | O | C-H | C-H |
| 1.10 | H | C | C | N-H | C-H | C-H |
| 1.11 | H | C | C | N-Me | C-H | C-H |
| 1.12 | H | C | C | C-H | N | O |
| 1.13 | H | C | C | C-H | O | N |
| 1.14 | H | C | C | N | O | C-H |
| 1.15 | H | C | C | O | N | C-H |
| 1.16 | H | C | C | C-H | N | S |
| 1.17 | H | C | C | C-H | S | N |
| 1.18 | H | C | C | N | S | C-H |
| 1.19 | H | C | C | S | N | C-H |
| 1.20 | H | C | C | O | C-H | N |
| 1.21 | H | C | C | N | C-H | O |
| 1.22 | H | C | C | S | C-H | N |
| 1.23 | H | C | C | N | C-H | S |
| 1.24 | H | C | C | C-H | N | N-H |
| 1.25 | H | C | C | C-H | N | N-Me |
| 1.26 | H | C | C | N | C-H | N-H |
| 1.27 | H | C | C | N | C-H | N-Me |
| 1.28 | H | C | C | N-H | C-H | N |
| 1.29 | H | C | C | N-Me | C-H | N |
| 1.30 | H | C | C | N | N-H | C-H |
| 1.31 | H | C | C | N | N-Me | C-H |
| 1.32 | H | C | C | N-Me | N | N |
| 1.33 | H | C | C | N-H | N | N |
| 1.34 | H | C | C | N | N | N-Me |
| 1.35 | H | C | C | N | N | N-H |
| 1.36 | H | C | N | N | N | N |
| 2.00 | H | C | C | C-H | C-Me | S |
| 2.01 | H | C | C | C-H | C-Me | O |
| 2.02 | H | C | C | C-Me | C-H | S |
| 2.03 | H | C | C | C-Me | C-H | O |
| 2.04 | H | C | C | C-H | C-Me | N-H |
| 2.05 | H | C | C | C-H | C-Me | N-Me |
| 2.06 | H | C | C | S | C-H | C-Me |
| 2.07 | H | C | C | O | C-H | C-Me |
| 2.08 | H | C | C | N-H | C-H | C-Me |
| 2.09 | H | C | C | N-Me | C-H | C-Me |
| 2.10 | H | C | C | S | C-Me | C-H |
| 2.11 | H | C | C | O | C-Me | C-H |
| 2.12 | H | C | C | N-H | C-Me | C-H |
| 2.13 | H | C | C | N-Me | C-Me | C-H |
| 2.14 | H | C | C | C-Me | N | O |
| 2.15 | H | C | C | C-Me | O | N |
| 2.16 | H | C | C | N | O | C-Me |
| 2.17 | H | C | C | O | N | C-Me |
| 2.18 | H | C | C | C-Me | N | S |
| 2.19 | H | C | C | C-Me | S | N |
| 2.20 | H | C | C | N | S | C-Me |
| 2.21 | H | C | C | S | N | C-Me |
| 2.22 | H | C | C | O | C-Me | N |
| 2.23 | H | C | C | N | C-Me | O |
| 2.24 | H | C | C | S | C-Me | N |
| 2.25 | H | C | C | N | C-Me | S |
| 2.26 | H | C | C | C-Me | N | N-H |
| 2.27 | H | C | C | C-Me | N | N-Me |
| 2.28 | H | C | C | N | C-Me | N-H |
| 2.29 | H | C | C | N | C-Me | N-Me |
| 2.30 | H | C | C | N-H | C-Me | N |
| 2.31 | H | C | C | N-Me | C-Me | N |
| 2.32 | H | C | C | N | N-H | C-Me |
| 2.33 | H | C | C | N | N-Me | C-Me |
| 3.00 | Me | C | C | C-H | C-H | S |
| 3.01 | Me | C | C | C-H | C-H | O |
| 3.02 | Me | C | C | C-H | C-H | N-H |
| 3.03 | Me | C | C | C-H | C-H | N-Me |
| 3.04 | Me | C | C | C-H | S | C-H |
| 3.05 | Me | C | C | C-H | O | C-H |
| 3.06 | Me | C | C | C-H | N-H | C-H |
| 3.07 | Me | C | C | C-H | N-Me | C-H |
| 3.08 | Me | C | C | S | C-H | C-H |
| 3.09 | Me | C | C | O | C-H | C-H |
| 3.10 | Me | C | C | N-H | C-H | C-H |
| 3.11 | Me | C | C | N-Me | C-H | C-H |
| 3.12 | Me | C | C | C-H | N | O |
| 3.13 | Me | C | C | C-H | O | N |
| 3.14 | Me | C | C | N | O | C-H |
| 3.15 | Me | C | C | O | N | C-H |
| 3.16 | Me | C | C | C-H | N | S |
| 3.17 | Me | C | C | C-H | S | N |
| 3.18 | Me | C | C | N | S | C-H |
| 3.19 | Me | C | C | S | N | C-H |
| 3.20 | Me | C | C | O | C-H | N |
| 3.21 | Me | C | C | N | C-H | O |
| 3.22 | Me | C | C | S | C-H | N |
| 3.23 | Me | C | C | N | C-H | S |
| 3.24 | Me | C | C | C-H | N | N-H |
| 3.25 | Me | C | C | C-H | N | N-Me |
| 3.26 | Me | C | C | N | C-H | N-H |
| 3.27 | Me | C | C | N | C-H | N-Me |
| 3.28 | Me | C | C | N-H | C-H | N |
| 3.29 | Me | C | C | N-Me | C-H | N |
| 3.30 | Me | C | C | N | N-H | C-H |
| 3.31 | Me | C | C | N | N-Me | C-H |
| 3.33 | Me | C | C | N-Me | N | N |
| 3.34 | Me | C | C | N-H | N | N |
| 3.35 | Me | C | C | N | N | N-Me |
| 3.36 | Me | C | C | N | N | N-H |
| 3.37 | Me | C | N | N | N | N |

The compounds of Formula (I) according to the invention can be used as plant growth regulators or seed germination promoters by themselves, but they are generally formulated into plant growth regulation or seed germination promotion compositions using formulation adjuvants, such as carriers, solvents and surface-active agents (SFAs). Thus, the present invention further provides a plant growth regulator composition comprising a plant growth regulation compound of Formula (I) and an agriculturally acceptable formulation adjuvant. The present invention further provides a seed germination promoter composition comprising a seed germination promoter compound of Formula (I) and an agriculturally acceptable formulation adjuvant. The composition can be in the form of concentrates which are diluted prior to use, although ready-to-use compositions can also be made. The final dilution is usually made with water, but can be made instead of, or in addition to, water, with, for example, liquid fertilisers, micronutrients, biological organisms, oil or solvents.

The compositions generally comprise from 0.1 to 99 % by weight, especially from 0.1 to 95 % by weight, compounds of Formula (I) and from 1 to 99.9 % by weight of a formulation adjuvant which preferably includes from 0 to 25 % by weight of a surface-active substance.

The compositions can be chosen from a number of formulation types, many of which are known from the Manual on Development and Use of FAO Specifications for Plant Protection Products, 5th Edition, 1999. These include dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), microemulsions (ME), suspension concentrates (SC), aerosols, capsule suspensions (CS) and seed treatment formulations. The formulation type chosen in any instance will depend upon the particular purpose envisaged and the physical, chemical and biological properties of the compound of Formula (I).

Dustable powders (DP) may be prepared by mixing a compound of Formula (I) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of Formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of Formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of Formula (I) and one or more powdered solid diluents or carriers, or from pre-formed blank granules by absorbing a compound of Formula (I) (or a solution thereof, in a suitable agent) in a porous granular material (such as pumice, attapulgite clays, fuller's earth, kieselguhr, diatomaceous earths or ground corn cobs) or by adsorbing a compound of Formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of Formula (I) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of Formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment.

Preparation of an EW involves obtaining a compound of Formula (I) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SFAs, to produce spontaneously a thermodynamically stable isotropic liquid formulation. A compound of Formula (I) is present initially in either the water or the solvent/SFA blend. Suitable solvents for use in MEs include those hereinbefore described for use in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of Formula (I). SCs may be prepared by ball or bead milling the solid compound of Formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of Formula (I) may be dry milled and added to water, containing agents hereinbefore described, to produce the desired end product.

Aerosol formulations comprise a compound of Formula (I) and a suitable propellant (for example *n*-butane). A compound of Formula (I) may also be dissolved or dispersed in a suitable medium (for example water or a water miscible liquid, such as *n*-propanol) to provide compositions for use in non-pressurised, hand-actuated spray pumps.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of Formula (I) and, optionally, a carrier or diluent therefor. The polymeric shell may be produced by either an interfacial polycondensation reaction or by a coacervation procedure. The compositions may provide for controlled release of the compound of Formula (I) and they may be used for seed treatment. A compound of Formula (I) may also be formulated in a biodegradable polymeric matrix to provide a slow, controlled release of the compound.

The composition may include one or more additives to improve the biological performance of the composition, for example by improving wetting, retention or distribution on surfaces; resistance to rain on treated surfaces; or uptake or mobility of a compound of Formula (I). Such additives include surface active agents (SFAs), spray additives based on oils, for example certain mineral oils or natural plant oils (such as soy bean and rape seed oil), and blends of these with other bio-enhancing adjuvants (ingredients which may aid or modify the action of a compound of Formula (I)).

Wetting agents, dispersing agents and emulsifying agents may be SFAs of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di-*iso*propyl- and tri-*iso*propyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately diesters), for example the reaction between lauryl alcohol and tetraphosphoric acid; additionally these products may be ethoxylated), sulphosuccinamates, paraffin or olefine sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

The present invention still further provides a method for regulating the growth of plants in a locus, wherein the method comprises application to the locus of a plant growth regulating amount of a composition according to the present invention.

The present invention also provides a method for promoting the germination of seeds, comprising applying to the seeds, or to a locus containing seeds, a seed germination promoting amount of a composition according to the present invention.

The application is generally made by spraying the composition, typically by tractor mounted sprayer for large areas, but other methods such as dusting (for powders), drip or drench can also be used. Alternatively the composition may be applied in furrow or directly to a seed before or at the time of planting.

The compound of formula (I) or composition of the present invention may be applied to a plant, part of the plant, plant organ, plant propagation material or a surrounding area thereof.

In one embodiment, the invention relates to a method of treating a plant propagation material comprising applying to the plant propagation material a composition of the present invention in an amount effective to promote germination and/or regulate plant growth. The invention also relates to a plant propagation material treated with a compound of formula (I) or a composition of the present invention. Preferably, the plant propagation material is a seed.

The term "plant propagation material" denotes all the generative parts of the plant, such as seeds, which can be used for the multiplication of the latter and vegetative plant materials such as cuttings and tubers. In particular, there may be mentioned the seeds, roots, fruits, tubers, bulbs, and rhizomes.

Methods for applying active ingredients to plant propagation material, especially seeds, are known in the art, and include dressing, coating, pelleting and soaking application methods of the propagation material. The treatment can be applied to the seed at any time between harvest of the seed and sowing of the seed or during the sowing process. The seed may also be primed either before or after the treatment. The compound of formula (I) may optionally be applied in combination with a controlled release coating or technology so that the compound is released over time.

The composition of the present invention may be applied pre-emergence or post-emergence. Suitably, where the composition is being used to regulate the growth of crop plants, it may be applied pre or post-emergence, but preferably post-emergence of the crop. Where the composition is used to promote the germination of seeds, it may be applied pre-emergence.

The rates of application of compounds of Formula I may vary within wide limits and depend on the nature of the soil, the method of application (pre- or post-emergence; seed dressing; application to the seed furrow; no tillage application etc.), the crop plant, the prevailing climatic conditions, and other factors governed by the method of application, the time of application and the target crop. For foliar or drench application, the compounds of Formula I according to the invention are generally applied at a rate of from 1 to 2000 g/ha, especially from 5 to 1000 g/ha. For seed treatment the rate of application is generally between 0.0005 and 150 g per 100 kg of seed.

Plants in which the composition according to the invention can be used include crops such as cereals (for example wheat, barley, rye, oats); beet (for example sugar beet or fodder beet); fruits (for example pomes, stone fruits or soft fruits, such as apples, pears, plums, peaches, almonds, cherries, strawberries, raspberries or blackberries); leguminous plants (for example beans, lentils, peas or soybeans); oil plants (for example rape, mustard, poppy, olives, sunflowers, coconut, castor oil plants, cocoa beans or groundnuts); cucumber plants (for example marrows, cucumbers or melons); fibre plants (for example cotton, flax, hemp or jute); citrus fruit (for example oranges, lemons, grapefruit or mandarins); vegetables (for example spinach, lettuce, asparagus, cabbages, carrots, onions, tomatoes, potatoes, cucurbits or paprika); lauraceae (for example avocados, cinnamon or camphor); maize; rice; tobacco; nuts; coffee; sugar cane; tea; vines; hops; durian; bananas; natural rubber plants; turf or ornamentals (for example flowers, shrubs, broad-leaved trees or evergreens such as conifers). This list does not represent any limitation.

The invention may also be used to regulate the growth, or promote the germination of seeds of non-crop plants, for example to facilitate weed control by synchronizing germination.

Crops are to be understood as also including those crops which have been modified by conventional methods of breeding or by genetic engineering. For example, the invention may be used in conjunction with crops that have been rendered tolerant to herbicides or classes of herbicides (e.g. ALS-, GS-, EPSPS-, PPO-, ACCase- and HPPD-inhibitors). An example of a crop that has been rendered tolerant to imidazolinones, e.g. imazamox, by conventional methods of breeding is Clearfield® summer rape (canola). Examples of crops that have been rendered tolerant to herbicides by genetic engineering methods include e.g. glyphosate- and glufosinate-resistant maize varieties commercially available under the trade names RoundupReady® and LibertyLink®. Methods of rending crop plants tolerant to HPPD-inhibitors are known, for example from WO0246387; for example the crop plant is transgenic in respect of a polynucleotide comprising a DNA sequence which encodes an HPPD-inhibitor resistant HPPD enzyme derived from a bacterium, more particularly from *Pseudomonas fluorescens* or *Shewanella colwelliana,* or from a plant, more particularly, derived from a monocot plant or, yet more particularly, from a barley, maize, wheat, rice, *Brachiaria, Chenchrus, Lolium, Festuca, Setaria, Eleusine, Sorghum* or *Avena* species.

Crops are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK® (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO 03/052073 and EP-A-427 529. Examples of transgenic plants comprising one or more genes that code for an insecticidal resistance and express one or more toxins are KnockOut® (maize), Yield Gard® (maize), NuCOTIN33B® (cotton), Bollgard® (cotton), NewLeaf® (potatoes), NatureGard® and Protexcta®. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crops are also to be understood to include those which are obtained by conventional methods of breeding or genetic engineering and contain so-called output traits (e.g. improved storage stability, higher nutritional value and improved flavour).
Compounds and compositions of the present invention may be applied in combination with other active ingredients or products for use in agriculture, including insecticides, fungicides, herbicides, plant growth regulators, crop enhancing compounds, nutrients and biologicals. Examples of suitable mixing partners may be found in the Pesticide Manual, 15th edition (published by the British Crop Protection Council). Such mixtures may be applied to a plant, plant propagation material or plant growing locus either simultaneously (for example as a pre-formulated mixture or a tank mix), or sequentially in a suitable timescale. Co-application of pesticides with the present invention has the added benefit of minimising farmer time spent applying products to crops.

The compounds of the invention may be made by the following methods.

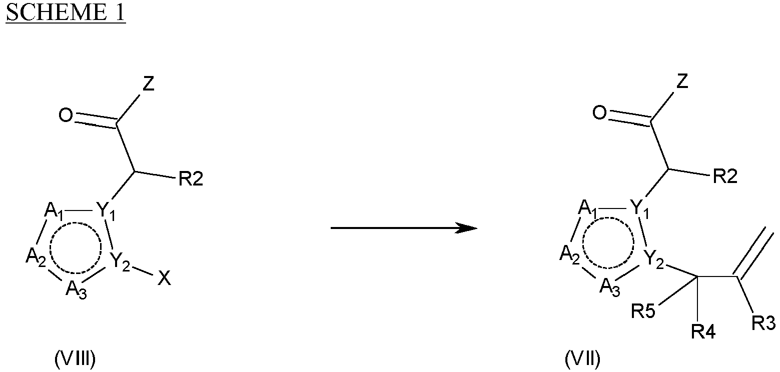

Compounds of Formula (VII), may be made by treatment of compounds of Formula (VIII) wherein Z is C1-C6-alkoxy (as for example in Macromolecules 2004, 37, 4351) and X is Br or I with a allyl derivative of formula MC(R4R5) C(R3)CH₂, wherein M is a boron or a tin derivatives in the presence of a suitable catalyst/ligand system, often a palladium (0) complex. 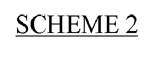 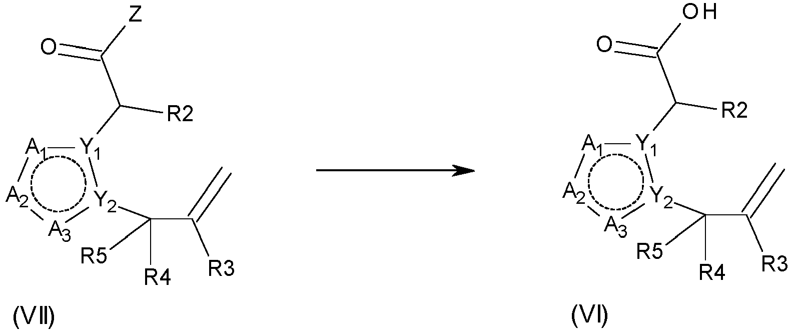 Compound of Formula (VI) may be prepared from compound of Formula (VII) wherein Z is C1-C6alkoxy by treatment with a base such as sodium hydroxyde in the presence of water.

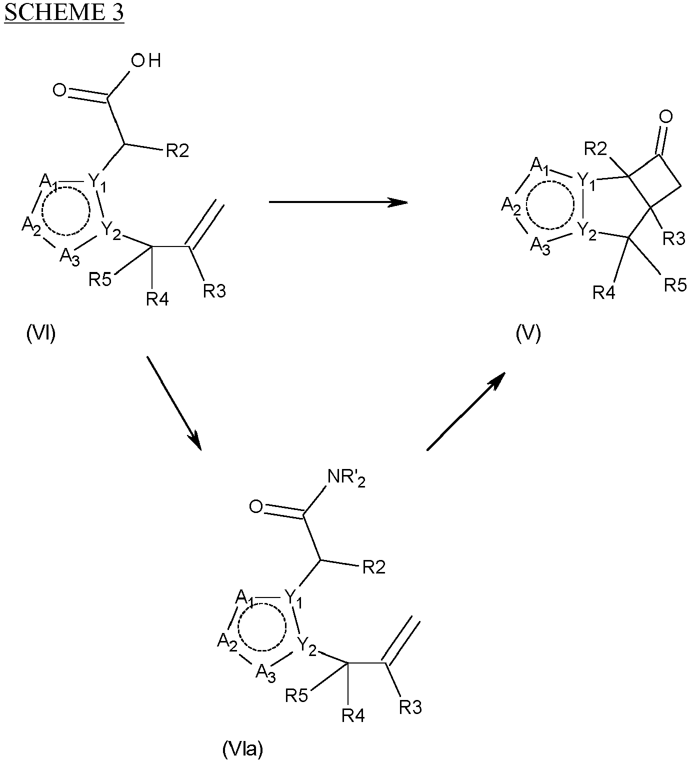

Compounds of Formula (V) may be made by treatment of compounds of Formula (VI) with a reagent used for the synthesis of acide chloride such as (1-chloro-2-methyl-propenyl)-dimethyl-amine, followed by reaction with a base such as triethylamine. The formation of acyl chloride is very well known, to a person skilled in the art and could be done with many other reagents such as thionyl chloride, oxaloyl chloride or phosphorus trichloride. The second reaction is known, to a person skilled in the art by processing via an intramolecular ketene cycloaddition.

Alternatively, compounds of Formula (V) may be made by treatment of compounds of Formula (VIa) wherein R' is a C1-C6alkyl with a dehydrating agent such as triflic anhydride in presence of a base such as collidine to give a iminium intermediate via intramolecular cycloaddition followed by hydrolysis with water. The use of compounds of Formula (VIa) wherein R' is chiral gives chiral compounds of Formula (V), (IV), (III), (II), (I).
Compounds of Formula (VIa) can be prepared from compounds of Formula (VI) with an amine of formula HNR'₂ wherein R' is not chiral such as pyrrolidine or R'₂ is chiral such as (*R,R*)-2,5-dimethylpyrrolidine. Such reactions may be carried out in the presence of a coupling reagent, such as DCC (N,N'-dicyclohexyl¬carbo¬diimide), EDC (1-ethyl-3-[3-dimethyl-amino-propyl]carbodiimide hydrochloride) or BOP-C1 (bis(2-oxo-3-oxazolidinyl)phosphonic chloride), in the presence of a base, such as pyridine, triethylamine, 4-(dimethylamino)pyridine or diisopropylethylamine, and optionally in the presence of a nucleophilic catalyst, such as hydroxybenzotriazole or 1-hydroxy-7-azabenzotriazole. Alternatively, this reaction can be performed in two steps by treatment of compound of formula (VI) with a reagent used for the synthesis of acide chloride followed by addition of an amine of formula HNR'₂ in the presence of a base such as triethylamine. 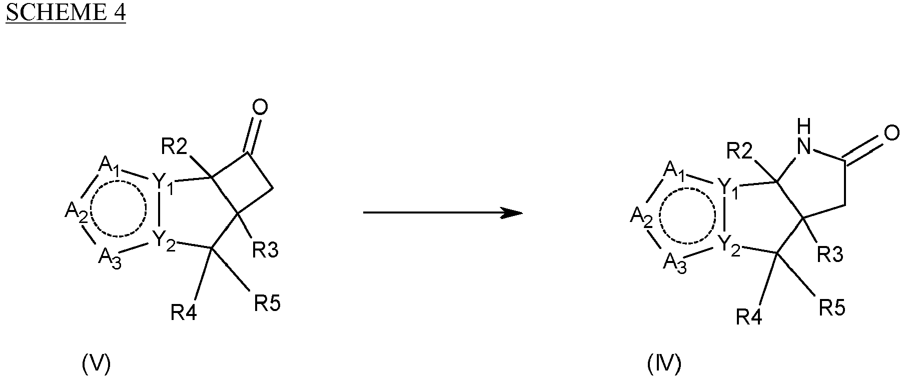 Compound of Formula (IV) can be pepared from compound of Formula (V) via formation of the oxime and rearrangement of the oxime by reaction for example with acetic anhydride or sulfonyl chloride. Alternatively, the compound of Formula (III) can be obtained directly by treatment with O-mesitylenesulfonylhydroxylamine. These reactions are known by a person skilled in the art as Beckmann rearrangement.

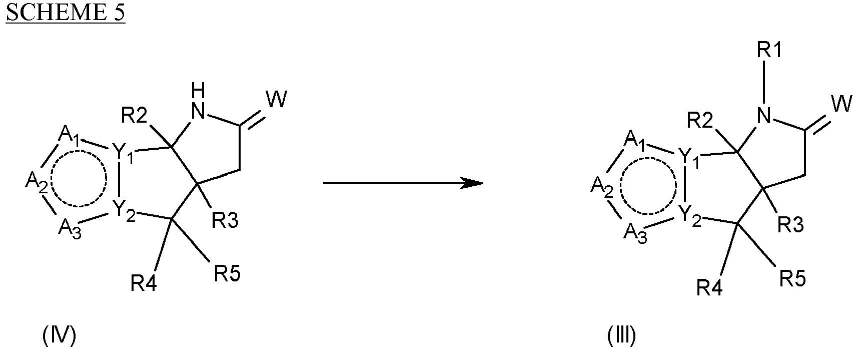

Compounds of Formula (III), wherein R1 is not hydrogen, may be prepared from a compound of Formula (IV) (wherein R1 is H) *via* alkylation by reaction of the amide with an alkylating agent such as an alkyl halide, in the presence of a base such as sodium hydride.

Compounds of Formula (III), wherein R1 is a carbonyl derivative, may be prepared by acylation of a compound of Formula (IV) with a compound of R'C(O)R, wherein R is OH, in the presence of a coupling reagent, such as DCC (N,N'-dicyclohexylcarbodiimide), EDC (1-ethyl-3-[3-dimethylamino-propyl]carbodiimide hydrochloride) or BOP-Cl (bis(2-oxo-3-oxazolidinyl)phosphonic chloride), in the presence of a base, such as pyridine, triethylamine, 4-(dimethylamino)pyridine or diisopropylethylamine, and optionally in the presence of a nucleophilic catalyst, such as hydroxybenzotriazole. Optionally, when R is Cl or OC(O)Cl-C6alkoxy, the acylation reaction may be carried out under basic conditions (for example in the presence of pyridine, triethylamine, 4-(dimethylamino)pyridine or diisopropylethylamine), optionally in the presence of a nucleophilic catalyst. Alternatively, the reaction may be conducted in a biphasic system comprising an organic solvent, preferably ethyl acetate, and an aqueous solvent, preferably a solution of sodium bicarbonate. Optionally, when R is C₁-C₆ alkoxy, the amide may be prepared by heating the derivative (IV) and amide (V) together. R' may be alkyl or alkoxy group.

Compounds of Formula (III), wherein R1 is an aromatic or heteroaromatic group, may be prepared from a compound of Formula (IV) by reaction of the amide with an aromatic or heteroaromatic compound of formula ArX, X being an halogen, in the presence of a base such as potassium phosphate and a suitable catalyst, often a copper (I) salt and a ligand such as dimethylethane-1,2-diamine.

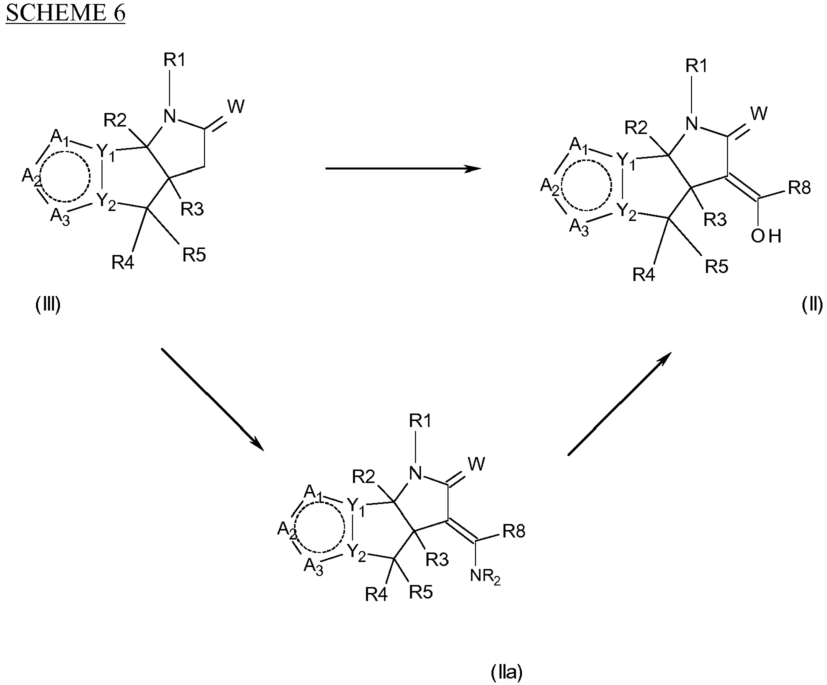

Compounds of Formula (II) may be prepared from a compound of Formula (III) via reaction with a formic ester derivative such as the methyl formate in presence of a base such as lithium diisopropylamide or potassium tert-butylate. Alternatively, compounds of Formula (II) may be prepared from a compound of Formula (IIa) via hydrolysis with an acid such as hydrogen chloride. Compounds of Formula (IIa) may be prepared from a compounds of Formula (III) via reaction with a Bredereck's reagent (t-butoxybis(dimethylamino)methane) wherein R is methyl or analogue.

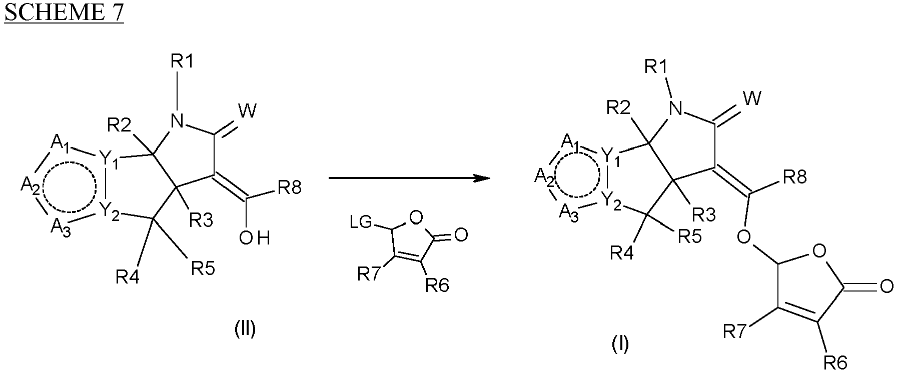

Compounds of Formula (I) may be prepared from a compounds of Formula (II) via nucleophilic substitution of a 5H-furanone derivative having a leaving group (LG) and LG is a leaving group, such as chlorine or bromine in presence of a base such as for example potassium tert-butylate, with or without a crown ether.

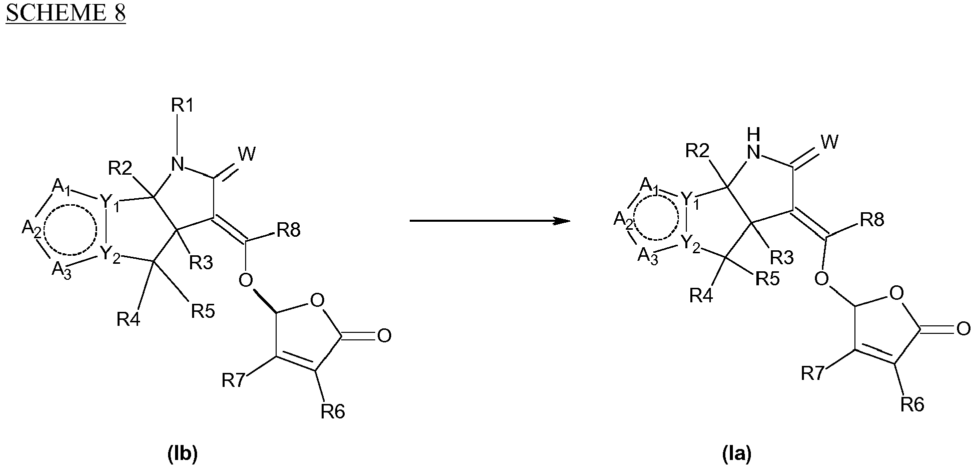

Alternatively, compounds of Formula (Ia), may be prepared from Formula (Ib) wherein R₁ is a protecting group such as tertbutoxycarbonyl by deprotection using an acid such as trifluoroacetic acid or hydrogen chloride or a Lewis acid such as magnesium chloride.

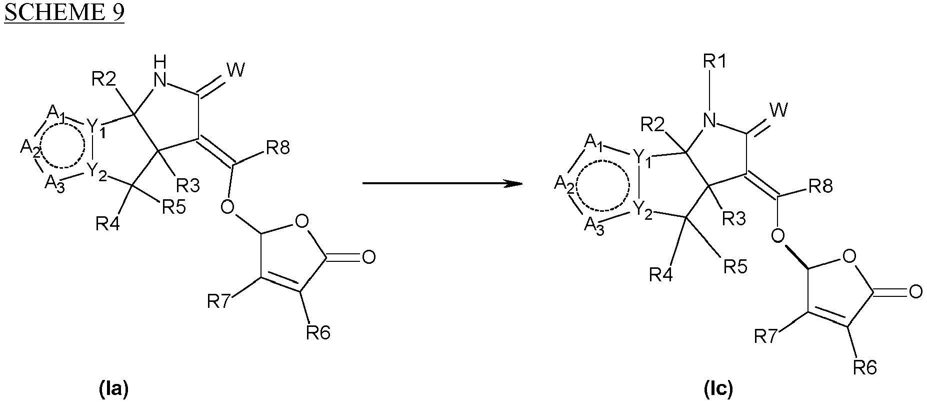

Compounds of Formula (Ic) wherein R1 is alkyl group, may be prepared from Formula (Ia) by reaction with an alkylating agent of formula R1X, wherein X is a leaving group such as halogen, tosyl or mesyl, in the presence of a base such as sodium hydride or silver oxide. Compounds of Formula (Ic) wherein R1 is an alkyl carbonyl or alkoxy carbonyl group can be prepared from compounds of Formula (Ia) by reaction with the corresponding acid chloride of formula R1Cl or the corresponding anhydride of formula R1₂O, in the presence of a base such as Hunig's base, triethyl amine or sodium carbonate, optionally in the presence of a nucleophile catalyst such as dimethylaminopyridine.

### EXAMPLES

The following HPLC-MS methods were used for the analysis of the compounds.
Method A: Spectra were recorded on a ZQ Mass Spectrometer from Waters (Single quadrupole mass spectrometer) equipped with an electrospray source (Polarity: positive or negative ions, Capillary: 3.00 kV, Cone: 30.00 V, Extractor: 2.00 V, Source Temperature: 100°C, Desolvation Temperature: 250°C, Cone Gas Flow: 50 L/Hr, Desolvation Gas Flow: 400 L/Hr, Mass range: 100 to 900 Da) and an Acquity UPLC from Waters (Solvent degasser, binary pump, heated column compartment and diode-array detector. Column: Waters UPLC HSS T3, 1.8 µm, 30 x 2.1 mm, Temp: 60 °C, flow rate 0.85 mL/min; DAD Wavelength range (nm): 210 to 500) Solvent Gradient: A = H₂O + 5% MeOH + 0.05 % HCOOH, B= Acetonitrile + 0.05 % HCOOH) gradient: 0 min 10% B; 0-1.2 min 100% B; 1.2-1.50 min 100% B
Method B: Spectra were recorded on a SQD Mass Spectrometer from Waters (Single quadrupole mass spectrometer) mass spectrometer equipped with an electrospray source (Polarity: positive and negative ions, Capillary: 3.00 kV, Cone: 30.00 V, Extractor: 2.00 V, Source Temperature: 150°C, Desolvation Temperature: 250°C, Cone Gas Flow: 0 L/Hr, Desolvation Gas Flow: 650 L/Hr, Mass range: 100 to 900 Da) and an Acquity UPLC from Waters (Binary pump, heated column compartment and diode-array detector, Solvent degasser, binary pump, heated column compartment and diode-array detector, Column: Waters UPLC HSS T3, 1.8 µm, 30 x 2.1 mm, Temp: 60 °C, flow rate 0.85 mL/min; DAD Wavelength range (nm): 210 to 500); Solvent Gradient: A = H₂O + 5% MeOH + 0.05 % HCOOH, B= Acetonitrile + 0.05 % HCOOH; gradient: 0 min 10% B; 0-1.2 min 100% B; 1.2-1.50 min 100% B

The following abbreviations are used throughout this section: s = singlet; bs = broad singlet; d = doublet; dd = double doublet; dt = double triplet; t = triplet, tt = triple triplet, q = quartet, m = multiplet; Me = methyl; Et = ethyl; Pr = propyl; Bu = butyl; M.p. = melting point; DMF = N, N-dimethylformamide, THF = tetrahydrofuran.

### Step 1: Ethyl 2-(2-allyl-3-thienyl)acetate

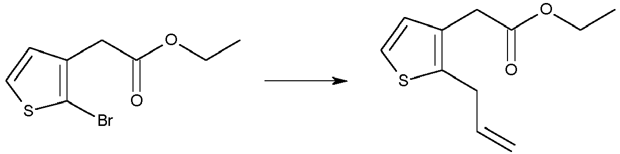

To a solution of ethyl 2-(2-bromo-3-thienyl)acetate (4.67 g, 18.7 mmol) in anhydrous DMF (75.0 mL) and the solution was degassed for 30 min. Lithium chloride (28.1 mmol, 1.19 g) was added followed by allyltri-n-butyltin (28.1 mmol, 9.60 g, 8.90 mL) and palladium triphenylphosphone tetrakis (1.31 mmol, 1.55 g) and the solution was heated to 100 °C for 4 h. Water (600 mL) and aqueous layer was extracted with ethyl acetate (3*300 mL). The combined organic layers were washed with brine (2*250 mL) dried and concentrated. The product was purified by flash chromatography (0% to 5% ethyl acetate in cyclohexane) to give ethyl 2-(2-allyl-3-thienyl)acetate (3.10 g, 79%). ¹H NMR (400 MHz, CDCl₃) 7.11 (1 H, d), 6.94 (1 H, d), 5.94 (1 H, m), 5.12 (1 H, m), 5.08 (1 H, t), 4.14 (2 H, q), 3.53 (4 H, m), 1.26 (3 H, t)

### Step 2: 2-(2-allyl-3-thienyl) acetic acid

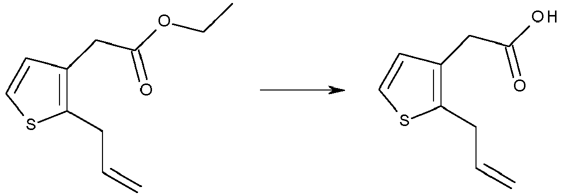

To a solution of ethyl 2-(2-allyl-3-thienyl)acetate (Step 1, 0.833 g, 3.96 mmol) in dioxane (4.5 mL) was added sodium hydroxide (4 M, 4.95 mmol, 1.24 mL) and the solution was stirred at 50 °C for 18 h. The solution was concentrated and water (50 mL) was added. The solution was extracted with diethyl ether (50 mL) and the pH of the aqueous layer was adjusted to 1. The aqueous layer was extracted with ethyl acetate (4*50 mL), washed with brine, dried and concentrated to give 2-(2-allyl-3-thienyl)acetic acid (670 mg, 93%). ¹H NMR (400 MHz, CDCl₃) 7.13 (1H, d), 6.94 (1 H, d), 5.94 (1 H, m), 5.12 (1 H, m), 5.08 (1 H, t), 3.61 (2 H, s), 3.58 (2 H, m).

### Step 3: 2-(2-allyl-3-thienyl)-1-pyrrolidin-1-yl-ethanone

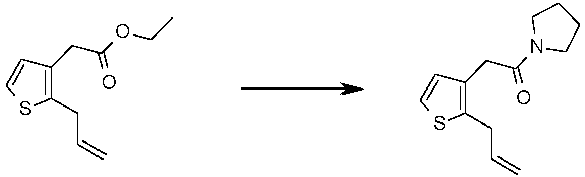

To a solution of 2-(2-allyl-3-thienyl)acetic acid (Step 2, 0.580 g, 3.18 mmol) in anhydrous DMF (13 mL), was added triethylamine (6.37 mmol, 0.896 mL), pyrrolidine (6.37 mmol, 0.537 mL), 1-ethyl-3-(3-dimethylaminopropyl)carbodiimide hydrochloride (4.77 mmol, 0.963 g) and hydroxy-7-azabenzotriazole (1.50 equiv., 4.77 mmol, 0.663 g). The solution was stirred at room temperature overnight. HCl (1 M, 100 mL) was added and the solution was extracted with diethylether (4*75 mL). The combined organic layers were washed with brine, dried and concentrated. The product was purified by flash chromatography (15% to 100% ethyl acetate in cyclohexane) to give 2-(2-allyl-3-thienyl)-1-pyrrolidin-1-yl-ethanone as an orange oil (524 mg, 70%). ¹H NMR (400 MHz, CDCl₃) 7.10 (1 H, d), 6.91 (1 H, d), 5.94 (1 H, m), 5.12 (1 H, m), 5.08 (1 H, t), 3.55 (4 H, m), 3.49 (2 H, t), 3.40 (2 H, t), 1.90 (4 H, m).

### Step 4: 1,2a,7,7a-tetrahydro-2H-cyclobut[a]thioinden-2-one

### Method A

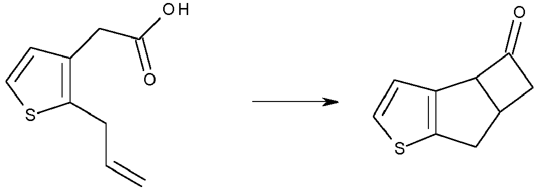

To a solution of 2-(2-allyl-3-thienyl)acetic acid (Step 2, 0.150 g, 0.823 mmol) in 1,2-dichloroethane (40 mL) was added 1-chloro-N,N,2-trimethyl-prop-1-en-1-amine (0.988 mmol, 0.132 g) at 0 °C. The solution as stirred for 1 h and then heated to reflux. Then triethylamine (0.99 mmol, 0.100 g) in dichloromethane (3 mL) was added over 1.5 h and the solution was stirred for another 2 h at 70 °C. The solution was cooled to room temperature and 50 mL of 1 M HCl were added. The solution was extracted with dichloromethane, dried and concentrated.
The product was purified by flash chromatography (0% to 15% ethyl acetate in cyclohexane) to give the title compound as an oil (41 mg, 30%). ¹H NMR (400 MHz, CDCl₃) 7.25 (1 H, d), 6.88 (1 H, d), 4.57 (1 H, m), 3.42 (1 H, m), 3.36 (2 H, m), 3.00 (2 H, m).

### Method B

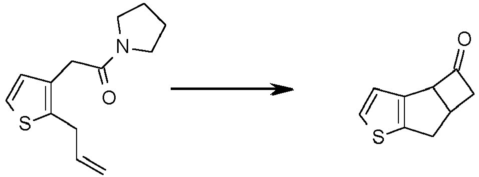

To a solution of 2-(2-allyl-3-thienyl)-1-pyrrolidin-1-yl-ethanone (Step 3, 1.692 g, 7.191 mmol) in dichloromethane (150 mL) was added collidine (12.94 mmol, 1.73 mL) and trifluoromethanesulfonic anhydride (10.0 mmol, 1.73 mL) in dichloromethane (10 mL). The solution was stirred for 5.5 h at room temperature and the solvents were removed. The residue was partitioned between carbon tetrachloride (4 mL) and water (4 mL) and the biphasic mixture was heated to 70 °C overnight. The aqueous layer was then extracted with dichloromethane and the combined organic layers were dried and concentrated. The product was purified by flash chromatography (0% to 15% ethyl acetate in cyclohexane) to give the title compound as an oil (895 mg, 76%). Analytical data were identical to method A.

### Step 5: 2-Oxo-3,3a,4,8b-tetrahydro-2H-thioindeno[1,2-b]pyrrole-1-carboxylic acid tert-butyl ester

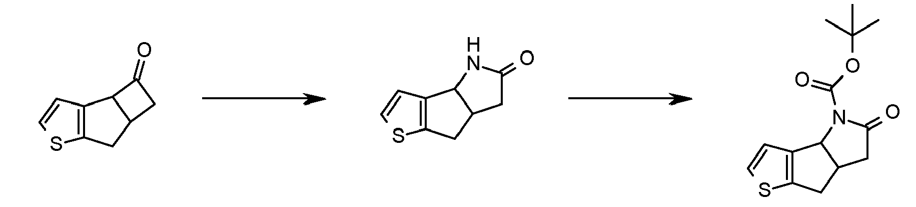

Step 5a: To a solution of 1,2a,7,7a-tetrahydro-2H-cyclobut[a]thioinden-2-one (Step 4, 5.45 mmol, 0.895 g) in dichloromethane (22 mL) was added *O*-mesitylenesulfonylhydroxylamine (7.09 mmol, 2.18 g) and the solution was stirred at room temperature for 6 h. Another 0.75 equiv of *O*-mesitylenesulfonylhydroxylamine was added after 5 h. The solvent were removed in vacuo and the residue was diluted with ethyl acetate and washed with sat. NaHCO₃, dried and concentrated to give the intermediate lactam as a white solid which was used without purification in the next step. ¹H NMR (400 MHz, DMSO-d*6*) 7.59 (1 H, brs), 6.97 (1 H, d), 6.63 (1 H, d), 4.65 (1 H, d), 3.00 (1 H, dd), 2.54-2.39 (3 H, m), 2.00 (1 H, d).
Step 5b: The previous residue (0.977 g) was taken up in dichloromethane (55 mL) and triethylamine (16.4 mmol, 2.29 mL), di-tert-butyl dicarbonate (16.4 mmol, 3.68 g) and 4-(*N,N*-dimethylamino)pyridine (1.09 mmol, 0.136 g) were added and the solution was stirred overnight at room temperature. Water was added and the aqueous layer was extracted with dichloromethane (4*20 mL). The combined organic layers were dried and concentrated. The product was purified by flash chromatography (5% ethyl acetate to 35% ethyl acetate in cyclohexane) to give title compound as a pale solid (950 mg, 62% over 2 steps). ¹H NMR (400 MHz, CDCl₃) 7.24 (1 H, d) 7.03 (1 H, d), 5.42 (1 H, d), 3.49 (1 H, m), 3.25 (1 H, dd), 2.90 (1 H, dd), 2.82 (1 H, d), 2.47 (1 H, dd), 1.61 (9 H).

### Step 6: 3-[1-Dimethylamino-meth-(Z)-ylidene]-2-oxo-3,3a,4,8b-tetrahydro-2H-thioindeno[1,2-b]pyrrole-1-carboxylic acid tert-butyl ester

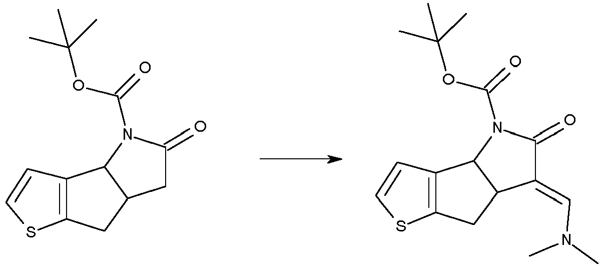

A solution of 2-oxo-3,3a,4,8b-tetrahydro-2H-thioindeno[1,2-b]pyrrole-1-carboxylic acid tert-butyl ester (Step 5, 0.533 mmol, 0.149 g) in 1-tert-butoxy-*N,N,N',N'-*tetramethylmethylenediamine (1.60 mmol, 0.33 mL) and toluene (3 mL) was heated to 100 °C for 3.5 h. the reaction mixture was cooled to room temperature and water was added. The aqueous layer was extracted with ethyl acetate (4*20 mL). The combined organic layers were dried and concentrated. The product was purified by flash chromatography (0% ethyl acetate to 50% ethyl acetate in cyclohexane) to give the title compound as a pale solid (132 mg, 74%). ¹H NMR (400 MHz, CDCl₃) 7.09 (1 H, s), 7.07 (1 H, d), 7.01 (1 H, d), 5.23 (1 H, d), 4.30 (1 H, m), 3.25 (1 H, dd), 2.98 (6 H, s), 2.68 (1 H, d), 1.61 (9 H, s).

### Step 7: 3-[1-Hydroxy-meth-(Z)-ylidene]-2-oxo-3,3a,4,8b-tetrahydro-2H-thioindeno[1,2-b]pyrrole-1-carboxylic acid tert-butyl ester

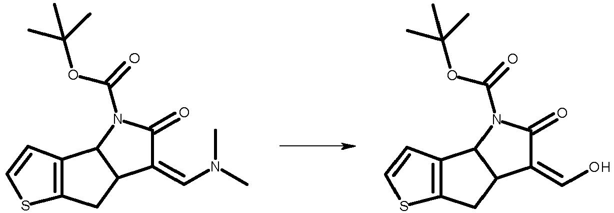

To a solution of 3-[1-dimethylamino-meth-(Z)-ylidene]-2-oxo-3,3a,4,8b-tetrahydro-2H-thioindeno[1,2-b]pyrrole-1-carboxylic acid tert-butyl ester (Step 6, 0.107 g, 0.32 mmol) in dioxane (5 mL) was added HCl (2 M, 0.48 mL). The solution was stirred at room temperature for 1.5 h and was then diluted with water, extracted with ethyl acetate, washed with brine, dried and concentrated to give the desired product (99 mg, quant.). ¹H NMR (400 MHz, CDCl₃) (mixture of enol and aldehyde) 11.12 (0.8 H), 7.87 (0.2 H, s), 7.25-6.90 (2.8 H, m), 5.48-5.32 (1.2 H, m), 4.12-3.95 (1 H, m), 3.35-3.25 (1 H, m), 2.82-2.69 (1 H, m), 1.60 (9 H, s).

### Step 8: Diastereoisomers of (3E)-3-[(2R*)(4-methyl-5-oxo-2H-furan-2-yl)oxymethylene]-3,3a,4,8b-tetrahydro-1H-thioindeno[1,2-b]pyrrol-2-one 1-carboxylic acid tert-butyl ester A1 and A2.

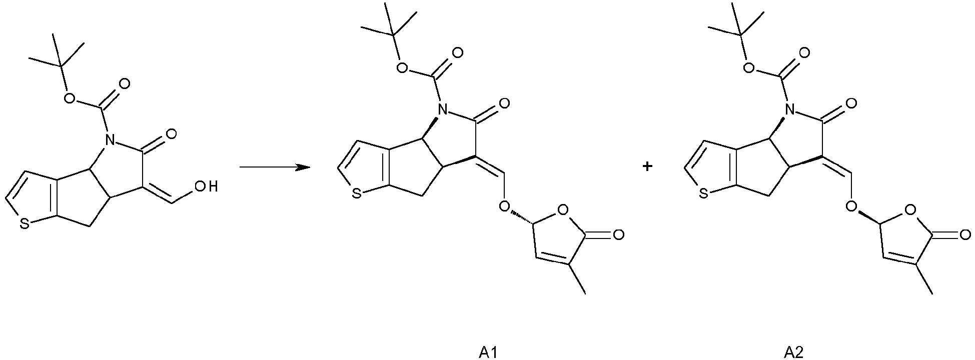

To a solution of 3-[1-hydroxy-meth-(Z)-ylidene]-2-oxo-3,3a,4,8b-tetrahydro-2H-thioindeno[1,2-b]pyrrole-1-carboxylic acid tert-butyl ester (Step 7, 0.099 g, 0.32 mmol) in THF (4.0 mL) cooled to 0 °C was added potassium tert-butoxide (0.483 mmol, 0.0553 g) and 18-crown-6-ether (0.483 mmol, 0.129 g). After 10 min, add 2-chloro-4-methyl-2H-furan-5-one (0.386 mmol, 0.0512 g)(as prepared in J. Chem. Soc., Perkin Trans. 1, 1981, 1734-1743) in 1 mL of THF and the solution was stirred for 40 min. Water was added and the aqueous layer was extracted with ethyl acetate (4*20 mL). The combined organic layers were dried and concentrated. The product was purified by flash chromatography (20% ethyl acetate to 50% ethyl acetate in cyclohexane) to give the desired product as two isomers:
- less polar diastereoisomer: **(3aR*,8bS*)-(3E)-3-[(2R*)(4-methyl-5-oxo-2H-furan-2-yl)oxymethylene]-3,3a,4,8b-tetrahydro-1H-thioindeno[1,2-b]pyrrol-2-one 1-carboxylic acid tert-butyl ester A1** (54 mg, 42%). ¹H NMR (400 MHz, CDCl₃) 7.44 (1 H, d), 7.20 (1 H, d), 7.04 (1 H, d), 6.95 (1 H, s), 6.17 (1 H, s), 5.44 (1 H, d), 4.12 (1 H, m), 3.36 (1 H, dd), 3.05 (1 H, d), 2.02 (3 H, s), 1.60 (9 H, s); LCMS (Method A) RT= 1.05 min; ES+ 326 (M+Na⁺);
- more polar diastereoisomer: **(3aR*,8bS*)-(3E)-3-[(2S*)(4-methyl-5-oxo-2H-furan-2-yl)oxymethylene]-3,3a,4,8b-tetrahydro-1H-thioindeno[1,2-b]pyrrol-2-one 1-carboxylic acid tert-butyl ester A2** (32 mg, 24%). ¹H NMR (400 MHz, CDCl₃) 7.44 (1 H, d), 7.19 (1 H, d), 7.03 (1 H, d), 6.99 (1 H, s), 6.18 (1 H, s), 5.44 (1 H, d), 4.11 (1 H, m), 3.36 (1 H, dd), 3.05 (1 H, d), 2.03 (3 H, s), 1.60 (9 H, s). LCMS (Method A) RT= 1.05 min; ES+ 326 (M+Na⁺)

### -tert-butyl (3E)-3-[(3,4-dimethyl-5-oxo-2H-furan-2-yl)oxymethylene]2-oxo-3,3a,4,8b-tetrahydro-2H-thioindeno[1,2-b]pyrrole-carboxylate A5

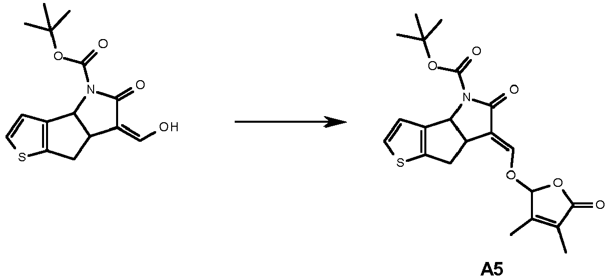

The compound was prepared in a similar manner to A1 and A2 starting from 3-[1-hydroxymeth-(Z)-ylidene]-2-oxo-3,3a,4,8b-tetrahydro-2H-thioindeno[1,2-b]pyrrole-1-carboxylic acid tert-butyl ester and 5-chloro-3,4-dimethyl-2(5H)-furanone (as prepared in Tetrahedron 1978, 34(13), 1935-42)). The compound tert-butyl (3E)-3-[(3,4-dimethyl-5-oxo-2H-furan-2-yl)oxymethylene] 2-oxo-3,3a,4,8b-tetrahydro-2H-thioindeno[1,2-b]pyrrole-carboxylate A5 was obtained as a mixture of diastereoisomers. LCMS (Method A) RT= 1.09 min; ES+ 481 (M+MeCN+Na⁺).

### Step 9: diastereoisomer of (3aR*,8bS*)-(3E)-3-[(2R)(4-methyl-5-oxo-2H-furan-2-yl)oxymethylene]-3,3a,4,8b-tetrahydro-1H-thioindeno[1,2-b]pyrrol-2-one A3

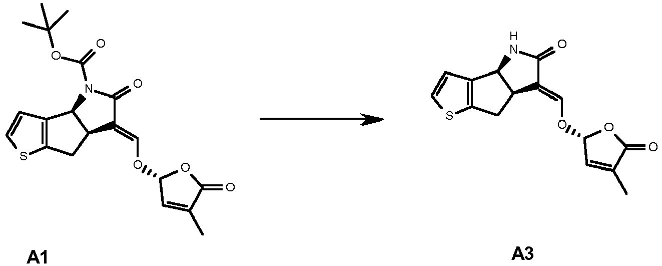

A solution of **A1** (Step 8, less polar isomer, 55 mg, 0.135 mmol) and magnesium chloride (20 mg, 0.203 mmol) in acetonitrile (2.0 mL) was stirred overnight at 40 °C. The solution was diluted with ethyl acetate and filtered and the filtrate was evaporated. The product was purified by flash chromatography (ethyl acetate) to give the desired product **A3** (6.9 mg, 29%). ¹H NMR (400 MHz, CD₂Cl₂) 7.25 (1 H, d), 7.21 (1 H, d), 6.95 (1 H, brs), 6.93 (1 H, s), 6.85 (1 H, d), 6.14 (1 H, s), 4.94 (1 H, d), 4.26 (1 H, m), 3.37 (1 H, dd), 2.98 (1 H, d), 1.98 (3 H, s). LCMS (Method B) RT= 0.80 min; ES+ 304 (M+H⁺);

### Diastereoisomer of (3aR*,8bS*)-(3E)-3-[(2S*)(4-methyl-5-oxo-2H-furan-2-yl)oxymethylene]3,3a,4,8b-tetrahydro-2H-thioindeno[1,2-b]pyrrol-2-one A4

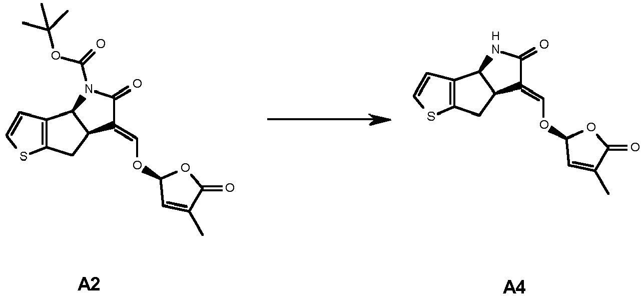

The product **A4** was obtained from **A2** using a similar procedure to **A3**. ¹H NMR (400 MHz, CD₂Cl₂) 7.26 (1 H, d), 7.25 (1 H, d), 6.97 (1 H, s), 6.87 (1 H, d), 6.61 (1 H, brs), 6.16 (1 H, s), 4.98 (1 H, d), 4.29 (1 H, m), 3.37 (1 H, dd), 2.98 (1 H, d), 1.99 (3 H, s). LCMS (Method B) RT= 0.81 min; ES+ 304 (M+H⁺);

### Step 10: (3E)-1-acetyl-3-[(4-methyl-5-oxo-2H-furan-2-yl)oxymethylene]3,3a,4,8b-tetrahydro-2H-thioindeno[1,2-b]pyrrol-2-one A6.

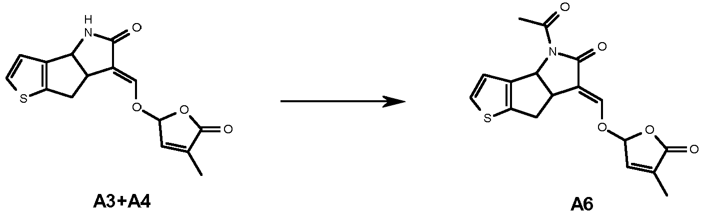

(3E)-3-[(4-methyl-5-oxo-2H-furan-2-yl)oxymethylene]-3,3a,4,8b-tetrahydro-1H-thioindeno[l,2-b]pyrrol-2-one **A3+A4** (20 mg, 0.065 mmol) and dimethylaminopyridine (1.6 mg, 0.013 mmol) were solved in dichloromethane (2 mL) and triethylamine (26 mg, 0.0371 mL, 0.263 mmol) was added. To the solution was added acetic anhydride (20 mg, 0.019 mL, 0.197 mmol) dropwise and the reaction mixture was stirred overnight at room temperature. The reaction mixture was poured in a separation funnel, diluted with ethyl acetate, washed with saturated NH₄Cl solution, water and brine, dried over MgSO₄ and the solvent was evaporated. The product was purified by flash chromatography (0-100% EA) to give (3E)-1-acetyl-3-[(4-methyl-5-oxo-2H-furan-2-yl)oxymethylene]3,3a,4,8b-tetrahydro-2H-thioindeno[1,2-b]pyrrol-2-one **A6** (16.5 mg, 72%). LCMS (Method A) RT= 0.94 min; ES+ 346 (M+H⁺);

**Table 3: Compounds of Formula (I)**

| | | | | |
|---|---|---|---|---|
| 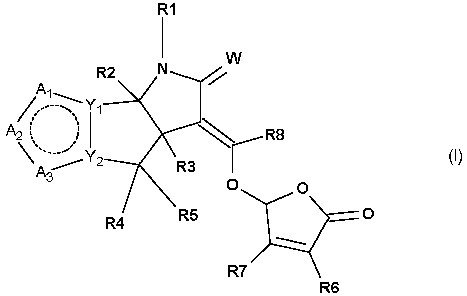 | | | | |

| **Example** | **Structure** | LCMS method | Retention (min.) | Mass |
|---|---|---|---|---|
| A1 | 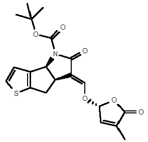 | A | 1.05 | 326 (M+Na⁺) |
| A2 | 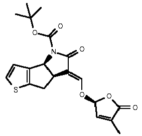 | A | 1.05 | 326 (M+Na⁺) |
| A3 | 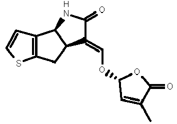 | B | 0.81 | 304 (M+H⁺) |
| A4 | 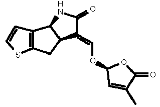 | B | 0.81 | 304 (M+H⁺); |
| A5 | 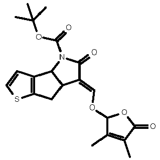 | A | 1.09 | 481 (M+MeCN+Na⁺) |
| A6 | 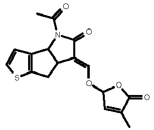 | A | 0.94 | 346 (M+H⁺) |

### Biological examples

The effect of compounds of Formula (I) on germination of *Orobanche cumana* Wallr. seeds was evaluated on glass fiber filter paper (GFFP) in petri dishes. Seeds were preconditioned at moisture and suitable temperature to become responsive to the specific chemical germination stimulants.

Test compounds were dissolved in DMSO (10 000mg1⁻¹) and stored at room temperature in a desiccators with desiccants. The stock solutions were dissolved with deionised water to the appropriate final test concentration.

Seeds of *O*. *cumana* race 'F' were collected from sunflower fields in Manzanilla (Seville, Spain) in 2008 (seed lot IN153) and stored at room temperature. To separate seeds from heavy organic debris, a modified sucrose floatation technique as described by Hartman & Tanimonure (Plant Disease (1991), 75, p.494) was applied. Seeds were filled into a separation funnel and stirred in water. When seeds floated to the surface, the water fraction containing heavy debris was discarded. Seeds were re-suspended in 2.5M sucrose solution (specific gravity of 1.20) and heavy debris was allowed to settle down for 60min. After removing debris, seeds were disinfected in 1% sodium hypochlorite solution and 0.025% (v/v) Tween 20 for 2min. The seeds were decanted onto two layers of cheesecloth, rinsed with sterile deionised water and re-suspended in sterile deionised water. Two ml of the seed suspension containing approximately 150-400 seeds were spread evenly on two layers of sterile glass fiber filter paper disc (Ø 9 mm) in Petri dishes (Ø 9 cm). After wetting the discs with 3ml sterile deionised water, petri dishes were sealed with parafilm. Seeds were incubated for 10 days at 20°C in the dark for seed conditioning. The upper disc with conditioned seeds was briefly dried, transferred to a petri dish lined with a dry GFFP disc, and wetted with 6ml of the appropriate test solution. The compounds of Formula (I) were tested at concentrations of 0.001, 0.01, and 0.1 mg 1⁻¹. The strigolactone analogue GR24 (commercially available as a mixture of isomers) was included as positive control and 0.001% DMSO as negative control. All treatments were tested in five replicates. Seeds were re-incubated at 20°C in the dark and examined for germination 10 days later. The radicles of germinated seeds were stained for 5min with blue ink (MIGROS, Switzerland) in 5% acetic acid according to Long et al. (Seed Science Research (2008), 18, p. 125). After staining, seeds were scanned using a flatbed scanner with an optical resolution of 1200 dpi (PULSTEK, OpticPro ST28) or photographed using a camera stand mounted with a digital SLR camera (Canon EOS 5D). Germination of 100 seeds per replicate was evaluated on digital images. Seeds were considered germinated when the radicle protruded from the seed coat. The results of the *Orobanche* seed germination tests are shown in Tables 4.

**Table 4: Effect of strigolactone analogs on germination of preconditioned Orobanche cumana seeds.**

| | Concentration (mg 1⁻¹) | | |
|---|---|---|---|
| Compound | 0.1 | 0.01 | 0.001 |
| | - Germination (%)* - | | |
| A3 | 84.6 | 85.6 | 87.8 |
| GR-24 | 78.4 | 65 | 67 |

| | | | |
|---|---|---|---|
| * Mean; N = 5 x 100 seeds; Seed lot IN153 Control, DMSO 0.01% (w/w): 1.4% germination | | | |

### Biological examples

The effect of compounds of Formula (I) on the germination of *Brassica oleracea* cv *Botrytis* or common cauliflower was tested on two types of cauliflowers: temperate types and tropical types. These two types were chosen because they display different sensitivities to the light conditions and temperature during germination. Germination of a sensitive temperate type is inhibited by light at 10°C while for the tropical types germination at 20° is stimulated by the presence of light. Hence, 10°C in the light and 20°C in the dark are considered suboptimal or stress conditions for germination of the two types, respectively.

The temperate seed batches tested are part of commercially produced seed batches of various varieties which are known to be sensitive to light at 10°C. These seeds were harvested and cleaned according standard commercial procedures. Ready seed batches were used (Ready indicates the processing level of these seeds: they have been cleaned and sized but received no other treatments). The tropical seed batches tested are part of seed batches produced as basic seed (for maintenance of the parental line) and were processed accordingly.

Germination was assessed using the standard paper germination test for Brassica: Fifty seeds were placed on blue germination paper, which was moistened with the appropriate solutions, in closed oblong germination boxes. Each condition was *tested in duplo*. Germination boxes were placed in controlled germination cabinets with the appropriate temperature and light conditions. Germination of seeds was counted at regular intervals. Seeds were considered to be germinated when the radical had protruded the testa and endosperm (radical size approximately 1 mm).

Test compounds were dissolved in DMSO at a concentration of 50 mM and stored at -20°C. The strigolactone analogue GR24 (commercially available as a racemic mixture of 2 diastereoisomers, referred to as "synthetic strigolactone GR-24" and first prepared by Johnson A. W. & all, Journal of the Chemical Society, Perkin Transactions 1, 1981, page 1734-1743) was included as positive control. Germination solutions were prepared by diluting the stock solutions with demineralized water till 25 µM. As control solutions demineralized water and a 0.05% v/v DMSO solution were used.

The effect of the strigolactone derivatives on germination is shown in tables 5. These results show that strigolactones stimulate germination at suboptimal conditions.

**Table 5: Germination of seeds of the temperate cauliflower Spacestar (seed batch 11B313; produced in South Africa in 2010 (cold-sensitive) and seed batch 11B314; produced in Chile in 2010 (not cold sensitive)) in the presence of 25 µM of the different strigolactone derivatives at 10°C and in the light.**

| | Spacestar 11 B313 | | Spacestar 11 B314 | |
|---|---|---|---|---|
| | Gₘₐₓ^{a} | stimulation ^{b} | Gₘₐₓ^{a} | stimulation ^{b} |
| compound | | (%) | (%) | (%) |
| DMSO | 60.0 | 0.0 | 90.5 | 0.0 |
| GR24 | 71.5 | 19.2 | 93.5 | 3.3 |
| A3 | 75.0 | 38.9 | 94.0 | 9.3 |

| | | | | |
|---|---|---|---|---|
| ^{a}: total germination as percentage of sown seeds. ^{b}: extra germination compared to the DMSO treatment (control), expressed as percentage of the germination in the DMSO treatment | | | | |

## Claims

1. A compound of Formula (I) 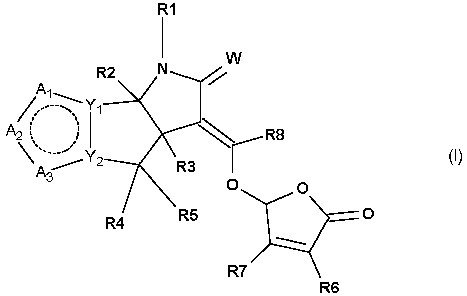 wherein
W is O or S;
R2 and R3 are independently hydrogen, or C₁-C₃ alkyl;
R4 and R5 are independently hydrogen, halogen, nitro, cyano, C₁-C₃ alkyl, C₁-C₃ haloalkyl, C₁-C₃ alkoxy, C₁-C₃ haloalkoxy, hydroxyl, -OC(O)R9, amine, N- C₁-C₃ alkyl amine, or N,N-di-C₁-C₃ alkyl amine;
R9 is hydrogen, C₁-C₆ alkyl, C₁-C₆ alkoxy, or C₁-C₆ haloalkyl;
R6 and R7 are independently hydrogen, C₁-C₃ alkyl, hydroxyl, halogen, or C₁-C₃ alkoxy;
R8 is hydrogen, nitro, cyano, C₁-C₆ alkyl, or C₁-C₆ haloalkyl;
R1 is hydrogen, C₁-C₆ alkoxy, hydroxyl, amine, N-C₁-C₆ alkyl amine, N,N-di- C₁-C₆ alkyl amine, C₁-C₆ alkyl optionally substituted by one to five R10, C₁-C₈ alkylcarbonyl-, or C₁-C₈ alkoxycarbonyl-, aryl optionally substituted by one to five R10, heteroaryl optionally substituted by one to five R10, heterocyclyl optionally substituted by one to five R10, benzyl optionally substituted by one to five R10, C₂-C₈ alkenyl optionally substituted by one to five R10, C₂-C₈ alkynyl optionally substituted by one to five R10, C₃-C₇ cycloalkyl, or C₃-C₁₀ cycloalkyl substituted by one to five R10;
R10 is cyano, nitro, halogen, hydroxyl, C₁-C₆ alkyl, C₁-C₆ alkoxy, C₁-C₆ haloalkoxy, C₁-C₆ haloalkyl, C₁-C₈ alkylthio-, C₁-C₈ haloalkylthio-, C₁-C₈ alkylsulfinyl-, N- C₁-C₆ alkyl amine, N,N-di- C₁-C₆ alkyl amine, C₁-C₈ haloalkylsulfinyl-, C₁-C₈ alkylsulfonyl-, C₁-C₈ haloalkylsulfonyl-, C₁-C₈alkylcarbonyl-, C₁-C₈ alkoxycarbonyl, C₂-C₆ alkenyl or C₂-C₆ alkynyl;
Y₁ and Y₂ are C;
A₁ and A₂ are C-X, wherein each X may be the same or different provided that A₁, A₂, A₃, Y₁ and Y₂ form an aromatic ring;
A₃ is sulphur;
and X is hydrogen, halogen, cyano, nitro, hydroxyl, C₁-C₆ alkoxy, C₁-C₆ haloalkoxy, amine, N- C₁-C₆ alkyl amine, N,N-di- C₁-C₆ alkyl amine, C₁-C₆ alkyl optionally substituted by one to five R10, C₁-C₈ alkylcarbonyl-, or C₁-C₈ alkoxycarbonyl-, N-C₁-C₆ alkyl aminocarbonyl, N,N-di C₁-C₆ alkyl aminocarbonyl, aryl optionally substituted by one to five R10, heteroaryl optionally substituted by one to five R10, heterocyclyl optionally substituted by one to five R10, C₂-C₈ alkenyl optionally substituted by one to five R10, C₂-C₈ alkynyl optionally substituted by one to five R10, C₃-C₇ cycloalkyl, C₁-C₁₀ cycloalkyl substituted by one to five R10, C₁-C₈ alkylthio-, C₁-C₈ haloalkylthio-, C₁-C₈ alkylsulfinyl-, C₁-C₈ haloalkylsulfinyl-, C₁-C₈ alkylsulfonyl-, or C₁-C₈ haloalkylsulfonyl;
or salts or N-oxides thereof.

2. A compound according to claim 1, wherein W is O.

3. A compound according to claim 2, wherein:
R2 and R3 are independently hydrogen, methyl, or ethyl;
R4 and R5 are independently hydrogen, hydroxyl, methyl or ethyl;
R6, R7 and R8 are independently hydrogen, methyl or ethyl;
R1 is selected from the group consisting of hydrogen, C₁-C₆ alkyl optionally substituted by one to five R10, C₁-C₈ alkylcarbonyl-, or C₁-C₈ alkoxycarbonyl-, aryl optionally substituted by one to five R10, or heteroaryl optionally substituted by one to five R10,
heterocyclyl optionally substituted by one to five R10, benzyl optionally substituted by one to five R10;
R10 is independently cyano, nitro, halogen, C₁-C₆ alkyl, C₁-C₆ alkoxy, or C₁-C₆ haloalkyl;
Y₁ and Y₂ are carbon;
A₁, and A₂ are independently C-X;
X is hydrogen, hydroxyl, halogen, cyano, methyl, ethyl, n-propyl, hydroxymethyl,
trifluoromethyl or methoxy; and
A₃ is sulphur.

4. A compound according to claim 3, wherein R1 is hydrogen, C₁-C₆ alkyl optionally substituted by one to five R10, C₁-C₈ alkylcarbonyl-, C₁-C₈ alkoxycarbonyl-, benzyl optionally substituted by one to five R10, or aryl optionally substituted by one to five R10.

5. A compound according to claim 3, wherein X is hydrogen, methyl, methoxy or trifluoromethyl.

6. A plant growth regulator or seed germination promoting composition, comprising a compound according to any one of the preceding claims, and an agriculturally acceptable formulation adjuvant.

7. A method for regulating the growth of plants at a locus, wherein the method comprises applying to the locus a plant growth regulating amount of a compound according to claims 1 to 5, or a composition according to claim 6.

8. A method for promoting the germination of seeds comprising applying to the seeds, or a locus containing seeds, a seed germination promoting amount of a compound according to claims 1 to 5, or a composition according to claim 6.

9. Plant propagation material comprising a compound according to claims 1 to 5, or a composition according to claim 6.

10. A method for controlling weeds comprising applying to a locus containing weed seeds a seed germination promoting amount of a compound according to claims 1 to 5 or a composition according to claim 6, allowing the seeds to germinate, and then applying to the locus a post-emergence herbicide.

11. Use of a compound of Formula (I) as a plant growth regulator or a seed germination promoter.

12. A compound of Formula (II) 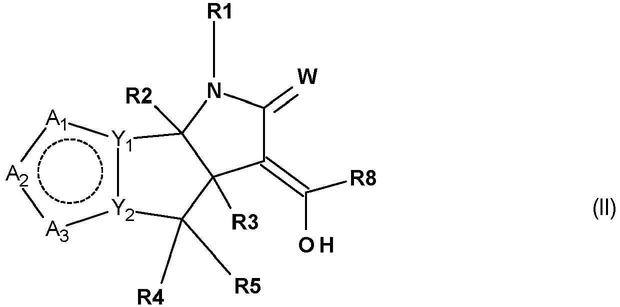 wherein
W is O or S;
R2 and R3 are independently hydrogen, or C1-C3 alkyl;
R4 and R5 are independently hydrogen, halogen, nitro, cyano, C₁-C₃ alkyl, C₁-C₃ haloalkyl, C₁-C₃ alkoxy, C₁-C₃ haloalkoxy, hydroxyl, -OC(O)R9, amine, N- C₁-C₃ alkyl amine, or N,N-di-C₁-C₃ alkyl amine;
R9 is hydrogen, C₁-C₆ alkyl, C₁-C₆ alkoxy, or C₁-C₆ haloalkyl;
R8 is hydrogen, nitro, cyano, C₁-C₆ alkyl, or C₁-C₆ haloalkyl;
R1 is hydrogen, C₁-C₆ alkoxy, hydroxyl, amine, N- C₁-C₆ alkyl amine, N,N-di- C₁-C₆ alkyl amine, C₁-C₆ alkyl optionally substituted by one to five R10, C₁-C₈ alkylcarbonyl-, or C₁-C₈ alkoxycarbonyl-, aryl optionally substituted by one to five R10, or heteroaryl optionally substituted by one to five R10, heterocyclyl optionally substituted by one to five R10, benzyl optionally substituted by one to five R10, C₂-C₈ alkenyl optionally substituted by one to five R10, C₂-C₈ alkynyl optionally substituted by one to five R10, C₃-C₇ cycloalkyl, or C₃-C₁₀ cycloalkyl substituted by one to five R10;
R10 is cyano, nitro, halogen, hydroxyl, C₁-C₆ alkyl, C₁-C₆ alkoxy, C₁-C₆ haloalkoxy, C₁-C₆ haloalkyl, C₁-C₈ alkylthio-, C₁-C₈ haloalkylthio-, C₁-C₈ alkylsulfinyl-, N- C₁-C₆ alkyl amine, N,N-di- C₁-C₆ alkyl amine, C₁-C₈ haloalkylsulfinyl-, C₁-C₈ alkylsulfonyl-, C₁-C₈ haloalkylsulfonyl-, C₁-C₈alkylcarbonyl-, C₁-C₈ alkoxycarbonyl, C₂-C₆ alkenyl, or C₂-C₆ alkynyl;
Y₁ and Y₂ are C;
A₁ and A₂ are each independently C-X, wherein each X may be the same or different, provided that A₁, A₂, A₃, Y₁ and Y₂ form an aromatic ring;
A₃ is sulphur;
and X is hydrogen, halogen, cyano, nitro, hydroxyl, C₁-C₆ alkoxy, C₁-C₆ haloalkoxy, amine, N- C₁-C₆ alkyl amine, N,N-di- C₁-C₆ alkyl amine, C₁-C₆ alkyl optionally substituted by one to five R10, C₁-C₈ alkylcarbonyl-, or C₁-C₈ alkoxycarbonyl-, N-C₁-C₆ alkyl aminocarbonyl, N,N-di C₁-C₆ alkyl aminocarbonyl, aryl optionally substituted by one to five R10, heteroaryl optionally substituted by one to five R10, heterocyclyl optionally substituted by one to five R10, C₂-C₈ alkenyl optionally substituted by one to five R10, C₂-C₈ alkynyl optionally substituted by one to five R10, C₃-C₇ cycloalkyl, C₁-C₁₀ cycloalkyl substituted by one to five R10, C₁-C₈ alkylthio-, C₁-C₈ haloalkylthio-, C₁-C₈ alkylsulfinyl-, C₁-C₈ haloalkylsulfinyl-, C₁-C₈ alkylsulfonyl-, or C₁-C₈ haloalkylsulfonyl;
or salts or N-oxides thereof.

## Patentansprüche

1. Verbindung der Formel (I), 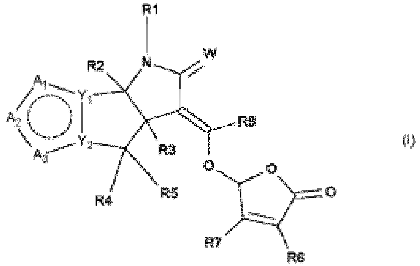 wobei
W O oder S ist;
R2 und R3 unabhängig Wasserstoff oder C₁-C₃-Alkyl sind; R4 und R5 unabhängig Wasserstoff, Halogen, Nitro, Cyano, C₁-C₃-Alkyl, C₁-C₃-Halogenalkyl, C₁-C₃-Alkoxy, C₁-C₃-Halogenalkoxy, Hydroxy, -OC(O)R9, Amin, N-C₁-C₃-Alkylamin oder N,N-Di-C₁-C₃-alkylamin sind;
R9 Wasserstoff, C₁-C₆-Alkyl, C₁-C₆-Alkoxy oder C₁-C₆-Halogenalkyl ist;
R6 und R7 unabhängig Wasserstoff, C₁-C₃-Alkyl, Hydroxy, Halogen oder C₁-C₃-Alkoxy sind;
R8 Wasserstoff, Nitro, Cyano, C₁-C₆-Alkyl oder C₁-C₆-Halogenalkyl ist;
R1 Wasserstoff, C₁-C₆-Alkoxy, Hydroxy, Amin, N-C₁-C₆-Alkylamin, N,N-Di-C₁-C₆-alkylamin, C₁-C₆-Alkyl, gegebenenfalls substituiert mit einem bis fünf R10, C₁-C₈-Alkylcarbonyl- oder C₁-C₈-Alkoxycarbonyl-, Aryl, gegebenenfalls substituiert mit einem bis fünf R10, Heteroaryl, gegebenenfalls substituiert mit einem bis fünf R10, Heterocyclyl, gegebenenfalls substituiert mit einem bis fünf R10, Benzyl, gegebenenfalls substituiert mit einem bis fünf R10, C₂-C₈-Alkenyl, gegebenenfalls substituiert mit einem bis fünf R10, C₂-C₈-Alkinyl, gegebenenfalls substituiert mit einem bis fünf R10, C₃-C₇-Cycloalkyl oder C₃-C₁₀-Cycloalkyl, substituiert mit einem bis fünf R10 ist;
R10 Cyano, Nitro, Halogen, Hydroxy, C₁-C₆-Alkyl, C₁-C₆-Alkoxy, C₁-C₆-Halogenalkoxy, C₁-C₆-Halogenalkyl, C₁-C₈-Alkylthio-, C₁-C₈-Halogenalkylthio-, C₁-C₈-Alkylsulfinyl-, N-C₁-C₆-Alkylamin, N,N-Di-C₁-C₆-alkylamin, C₁-C₈-Halogenalkylsulfinyl-, C₁-C₈-Alkylsulfonyl, C₁-C₈-Halogenalkylsulfonyl-, C₁-C₈-Alkylcarbonyl-, C₁-C₈-Alkoxycarbonyl, C₂-C₆-Alkenyl oder C₂-C₆-Alkinyl ist;
Y₁ und Y₂ C sind;
A₁ und A₂ C-X sind, wobei jedes X gleich oder verschieden sein kann, mit der Maßgabe, dass A₁, A₂, A₃, Y₁ und Y₂ einen aromatischen Ring bilden;
A₃ Schwefel ist;
und X Wasserstoff, Halogen, Cyano, Nitro, Hydroxy, C₁-C₆-Alkoxy, C₁-C₆-Halogenalkoxy, Amin, N-C₁-C₆-Alkylamin, N,N-Di-C₁-C₆-alkylamin, C₁-C₆-Alkyl, gegebenenfalls substituiert mit einem bis fünf R10, C₁-C₈-Alkylcarbonyl- oder C₁-C₈-Alkoxycarbonyl-, N-C₁-C₆-Alkylaminocarbonyl, N,N-Di-C₁-C₆-alkylaminocarbonyl, Aryl, gegebenenfalls substituiert mit einem bis fünf R10, Heteroaryl, gegebenenfalls substituiert mit einem bis fünf R10, Heterocyclyl, gegebenenfalls substituiert mit einem bis fünf R10, C₂-C₈-Alkenyl, gegebenenfalls substituiert mit einem bis fünf R10, C₂-C₈-Alkinyl, gegebenenfalls substituiert mit einem bis fünf R10, C₃-C₇-Cycloalkyl, C₃-C₁₀-Cycloalkyl, substituiert mit einem bis fünf R10, C₁-C₈-Alkylthio-, C₁-C₈-Halogenalkylthio-, C₁-C₈-Alkylsulfinyl-, C₁-C₈-Halogenalkylsulfinyl-, C₁-C₈-Alkylsulfonyl- oder C₁-C₈-Halogenalkylsulfonyl, ist;
oder Salze oder N-Oxide davon.

2. Verbindung gemäß Anspruch 1, wobei W O ist.

3. Verbindung gemäß Anspruch 2, wobei:
R2 und R3 unabhängig Wasserstoff, Methyl oder Ethyl sind;
R4 und R5 unabhängig Wasserstoff, Hydroxy, Methyl oder Ethyl sind;
R6, R7 und R8 unabhängig Wasserstoff, Methyl oder Ethyl sind;
R1 ausgewählt aus der Gruppe bestehend aus Wasserstoff, C₁-C₆-Alkyl, gegebenenfalls substituiert mit einem bis fünf R10, C₁-C₈-Alkylcarbonyl- oder C₁-C₈-Alkoxycarbonyl-, Aryl, gegebenenfalls substituiert mit einem bis fünf R10 oder Heteroaryl, gegebenenfalls substituiert mit einem bis fünf R10, Heterocyclyl, gegebenenfalls substituiert mit einem bis fünf R10, Benzyl, gegebenenfalls substituiert mit einem bis fünf R10, ist;
R10 unabhängig Cyano, Nitro, Halogen, C₁-C₆-Alkyl, C₁-C₆-Alkoxy oder C₁-C₆-Halogenalkyl ist;
Y₁ und Y₂ Kohlenstoff sind;
A₁ und A₂ unabhängig C-X sind;
X Wasserstoff, Hydroxy, Halogen, Cyano, Methyl, Ethyl, n-Propyl, Hydroxymethyl, Trifluormethyl oder Methoxy ist; und A₃ Schwefel ist.

4. Verbindung gemäß Anspruch 3, wobei R1 Wasserstoff, C₁-C₆-Alkyl, gegebenenfalls substituiert mit einem bis fünf R10, C₁-C₈-Alkylcarbonyl-, C₁-C₈-Alkoxycarbonyl-, Benzyl, gegebenenfalls substituiert mit einem bis fünf R10, oder Aryl, gegebenenfalls substituiert mit einem bis fünf R10, ist.

5. Verbindung gemäß Anspruch 3, wobei X Wasserstoff, Methyl, Methoxy oder Trifluormethyl ist.

6. Pflanzenwachstumsregulator- oder Samenkeimungsförderungs-Zusammensetzung, umfassend eine Verbindung gemäß einem der vorstehenden Ansprüche und ein landwirtschaftlich annehmbares Formulierungshilfsmittel.

7. Verfahren zum Regulieren des Wachstums von Pflanzen an einem Ort, wobei das Verfahren Aufbringen einer pflanzenwachstumsregulierenden Menge einer Verbindung gemäß Ansprüchen 1 bis 5 oder einer Zusammensetzung gemäß Anspruch 6 auf den Ort umfasst.

8. Verfahren zum Fördern der Keimung von Samen, umfassend Aufbringen einer samenkeimungsfördernden Menge einer Verbindung gemäß Ansprüchen 1 bis 5 oder einer Zusammensetzung gemäß Anspruch 6 auf die Samen oder einen Ort, der Samen enthält.

9. Pflanzenvermehrungsmaterial, umfassend eine Verbindung gemäß Ansprüchen 1 bis 5 oder eine Zusammensetzung gemäß Anspruch 6.

10. Verfahren zur Unkrautbekämpfung, umfassend Aufbringen einer samenkeimungsfördernden Menge einer Verbindung gemäß Ansprüchen 1 bis 5 oder einer Zusammensetzung gemäß Anspruch 6 auf einen Ort, der Unkrautsamen enthält, Keimenlassen der Samen und anschließend Aufbringen eines Nachlaufherbizids auf den Ort.

11. Verwendung einer Verbindung der Formel (I) als Pflanzenwachstumsregulator oder als Samenkeimungsförderer.

12. Verbindung der Formel (II) 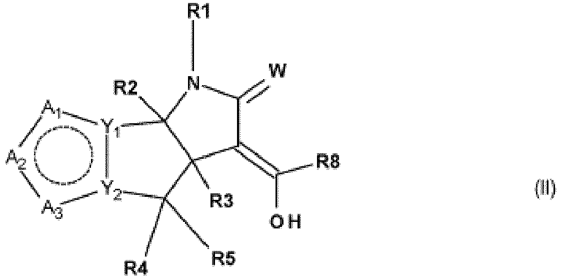 wobei
W O oder S ist;
R2 und R3 unabhängig Wasserstoff oder C₁-C₃-Alkyl sind;
R4 und R5 unabhängig Wasserstoff, Halogen, Nitro, Cyano, C₁-C₃-Alkyl, C₁-C₃-Halogenalkyl, C₁-C₃-Alkoxy, C₁-C₃-Halogenalkoxy, Hydroxy, -OC(O)R9, Amin, N-C₁-C₃-Alkylamin oder N,N-Di-C₁-C₃-alkylamin sind;
R9 Wasserstoff, C₁-C₆-Alkyl, C₁-C₆-Alkoxy oder C₁-C₆-Halogenalkyl ist;
R8 Wasserstoff, Nitro, Cyano, C₁-C₆-Alkyl oder C₁-C₆-Halogenalkyl ist;
R1 Wasserstoff, C₁-C₆-Alkoxy, Hydroxy, Amin, N-C₁-C₆-Alkylamin, N, N-Di-C₁-C₆-alkylamin, C₁-C₆-Alkyl, gegebenenfalls substituiert mit einem bis fünf R10, C₁-C₈-Alkylcarbonyl- oder C₁-C₈-Alkoxycarbonyl-, Aryl, gegebenenfalls substituiert mit einem bis fünf R10, oder Heteroaryl, gegebenenfalls substituiert mit einem bis fünf R10, Heterocyclyl, gegebenenfalls substituiert mit einem bis fünf R10, Benzyl, gegebenenfalls substituiert mit einem bis fünf R10, C₂-C₈-Alkenyl, gegebenfalls substituiert mit einem bis fünf R10, C₂-C₈-Alkinyl, gegebenenfalls substituiert mit einem bis fünf R10, C₃-C₇-Cycloalkyl oder C₃-C₁₀-Cycloalkyl, substituiert mit einem bis fünf R10 ist;
R10 Cyano, Nitro, Halogen, C₁-C₆-Alkyl, C₁-C₆-Alkoxy, C₁-C₆-Halogenalkoxy, C₁-C₆-Halogenalkyl, C₁-C₈-Alkylthio-, C₁-C₈-Halogenalkylthio-, C₁-C₈-Alkylsulfinyl-, N-C₁-C₆-Alkylamin, N,N-Di-C₁-C₆-alkylamin, C₁-C₈-Halogenalkylsulfinyl-, C₁-C₈-Alkylsulfonyl-, C₁-C₈-Halogenalkylsulfonyl-, C₁-C₈-Alkylcarbonyl-, C₁-C₈-Alkoxycarbonyl, C₂-C₆-Alkenyl oder C₂-C₆-Alkinyl ist;
Y₁ und Y₂ C sind;
A₁ und A₂ jeweils unabhängig C-X sind, wobei jedes X gleich oder verschieden sein kann, mit der Maßgabe, dass A₁, A₂, A₃, Y₁ und Y₂ einen aromatischen Ring bilden;
A₃ Schwefel ist;
und X Wasserstoff, Halogen, Cyano, Nitro, Hydroxy, C₁-C₆-Alkoxy, C₁-C₆-Halogenalkoxy, Amin, N-C₁-C₆-Alkylamin, N,N-Di-C₁-C₆-alkylamin, C₁-C₆-Alkyl, gegebenenfalls substituiert mit einem bis fünf R10, C₁-C₆-Alkylcarbonyl- oder C₁-C₈-Alkoxycarbonyl-, N-C₁-C₆-Alkylaminocarbonyl, N,N-Di-C₁-C₆-alkylaminocarbonyl, Aryl, gegebenenfalls substituiert mit einem bis fünf R10, Heteroaryl, gegebenenfalls substituiert mit einem bis fünf R10, Heterocyclyl, gegebenenfalls substituiert mit einem bis fünf R10, C₂-C₈-Alkenyl, gegebenenfalls substituiert mit einem bis fünf R10, C₂-C₈-Alkinyl, gegebenenfalls substituiert mit einem bis fünf R10, C₃-C₇-Cycloalkyl, C₃-C₁₀-Cycloalkyl, substituiert mit einem bis fünf R10, C₁-C₈-Alkylthio-, C₁-C₈-Halogenalkylthio-, C₁-C₈-Alkylsulfinyl-, C₁-C₈-Halogenalkylsulfinyl-, C₁-C₈-Alkylsulfonyl- oder C₁-C₈-Halogenalkylsulfonyl ist;
oder Salze oder N-Oxide davon.

## Revendications

1. Composé de Formule (I) 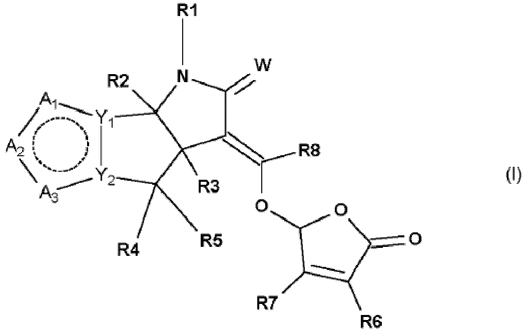 dans lequel
W est O ou S ;
R2 et R3 sont indépendamment hydrogène, ou C₁-C₃ alkyle ;
R4 et R5 sont indépendamment hydrogène, halogène, nitro, cyano, C₁-C₃ alkyle, C₁-C₃ halogénoalkyle, C₁-C₃ alcoxy, C₁-C₃-halogénoalcoxy, hydroxyle, -OC(O)R9, aminé, N-C₁-C₃ alkylamine, ou N,N-di-C₁-C₃ alkylamine ;
R9 est hydrogène, C₁-C₆ alkyle, C₁-C₆ alcoxy, ou C₁-C₆ halogénoalkyle ;
R6 et R7 sont indépendamment hydrogène, C₁-C₃ alkyle, hydroxyle, halogène ou C₁-C₃ alcoxy;
R8 est hydrogène, nitro, cyano, C₁-C₆ alkyle ou C₁-C₆ halogénoalkyle ;
R1 est hydrogène, C₁-C₆ alcoxy, hydroxyle, amine, N-C₁-C₆ alkylamine, N,N-di-C₁-C₆ alkylamine, C₁-C₆ alkyle éventuellement substitué par de un à cinq R10, C₁-C₈ alkylcarbonyle ou C₁-C₈ alcoxycarbonyle, aryle éventuellement substitué par de un à cinq R10, hétéroaryle éventuellement substitué par de un à cinq R10, hétérocyclyle éventuellement substitué par de un à cinq R10, benzyle éventuellement substitué par de un à cinq R10, C₂-C₈ alcényle éventuellement substitué par de un à cinq R10, C₂-C₈ alcynyle éventuellement substitué par de un à cinq R10, C₃-C₇ cycloalkyle, ou C₃-C₁₀ cycloalkyle éventuellement substitué par de un à cinq R10 ;
R10 est cyano, nitro, halogène, hydroxyle, C₁-C₆ alkyle, C₁-C₆ alcoxy, C₁-C₆ halogénoalcoxy, C₁-C₆ halogénoalkyle, C₁-C₈alkylthio-, C₁-C₈halogénoalkylthio-, C₁-C₈alkylsulfinyl-, N-C₁-C₆alkylamine, N,N-di-C₁-C₆alkylamine, C₁-C₈halogénoalkylsulfinyl-, C₁-C₈alkylsulfonyl-, C₁-C₈halogénoalkylsulfonyl-, C₁-C₈alkylcarbonyl-, C₁-C₈alcoxycarbonyle, C₂-C₆ alcényle, ou C₂-C₆ alcynyle ;
Y₁ et Y₂ sont C ;
A₁ et A₂ sont C-X, où chaque X peut être identique ou différent, à condition que A₁, A₂, A₃, Y₁ et Y₂ forment un cycle aromatique ;
A₃ est soufre ;
et X est hydrogène, halogène, cyano, nitro, hydroxyle, C₁-C₆ alcoxy, C₁-C₆halogénoalcoxy, amine, N-C₁-C₆alkylamine, N,N-di-C₁-C₆alkylamine, C₁-C₆alkyle éventuellement substitué par de un à cinq R10, C₁-C₈alkylcarbonyl- ou C₁-C₈alcoxycarbonyl-, N-C₁-C₆alkylaminocarbonyle, N,N-di-C₁-C₆alkylaminocarbonyle, aryle éventuellement substitué par de un à cinq R10, hétéroaryle éventuellement substitué par de un à cinq R10, hétérocyclyle éventuellement substitué par de un à cinq R10, C₂-C₈alcényle éventuellement substitué par de un à cinq R10, C₂-C₈alcynyle éventuellement substitué par de un à cinq R10, C₃-C₇cycloalkyle, C₃-C₁₀cycloalkyle substitué par de un à cinq R10, C₁-C₈alkylthio-, C₁-C₈halogénoalkylthio-, C₁-C₈alkylsulfinyl-, C₁-C₈halogénoalkylsulfinyl-, C₁-C₈alkylsulfonyl- ou C₁-C₈halogénoalkylsulfonyle ;
ou des sels ou des N-oxydes de celui-ci.

2. Composé selon la revendication 1, dans lequel W est O.

3. Composé selon la revendication 2, dans lequel
R2 et R3 sont indépendamment hydrogène, méthyle, ou éthyle ;
R4 et R5 sont indépendamment hydrogène, hydroxyle, méthyle ou éthyle ;
R6, R7 et R8 sont indépendamment hydrogène, méthyle ou éthyle ;
R1 est choisi dans le groupe constitué par hydrogène, C₁-C₆ alkyle éventuellement substitué par de un à cinq R10, C₁-C₈ alkylcarbonyle, ou C₁-C₈ alcoxycarbonyle, aryle éventuellement substitué par de un à cinq R10, ou hétéroaryle éventuellement substitué par de un à cinq R10, hétérocyclyle éventuellement substitué par de un à cinq R10, benzyle éventuellement substitué par de un à cinq R10 ; et
R10 est indépendamment cyano, nitro, halogène, C₁-C₆ alkyle, C₁-C₆ alcoxy, ou C₁-C₆ halogénoalkyle ;
Y₁ et Y₂ sont carbone ;
A₁ et A₂ sont indépendamment C-X ;
et
X est hydrogène, hydroxyle, halogène, cyano, méthyle, éthyle, n-propyle, hydroxyméthyle, trifluorométhyle ou méthoxy ; et
A₃ est soufre.

4. Composé selon la revendication 3, dans lequel R1 est hydrogène, C₁-C₆alkyle éventuellement substitué par de un à cinq R10, C₁-C₈alkylcarbonyl-, C₁-C₈alcoxycarbonyl-, benzyle éventuellement substitué par de un à cinq R10, ou aryle éventuellement substitué par de un à cinq R10.

5. Composé selon la revendication 3, dans lequel X est hydrogène, méthyle, méthoxy ou trifluorométhyle.

6. Composition régulatrice de croissance végétale ou favorisant la germination des graines, comprenant un composé selon l'une quelconque des revendications précédentes, et un adjuvant de formulation acceptable sur le plan agricole.

7. Méthode de régulation de la croissance de plantes au niveau d'un lieu, la méthode comprenant l'application au lieu d'une quantité régulatrice de croissance végétale d'un composé selon les revendications 1 à 5 ou d'une composition selon la revendication 6.

8. Méthode favorisant la germination de graines, comprenant l'application aux graines, ou à un lieu contenant des graines, d'une quantité favorisant la germination de graines d'un composé selon les revendications 1 à 5 ou d'une composition selon la revendication 6.

9. Matériel de propagation végétal, comprenant un composé selon les revendications 1 à 5, ou une composition selon la revendication 6.

10. Méthode de contrôle d'adventices, comprenant les étapes consistant à appliquer, à un lieu contenant des graines d'adventices, une quantité favorisant la germination de graines d'un composé selon les revendications 1 à 5 ou d'une composition selon la revendication 6, à permettre aux graines de germer, puis à appliquer au lieu un herbicide en post-émergence.

11. Utilisation d'un composé de Formule (I) comme régulateur de croissance végétale ou promoteur de germination de graines.

12. Composé de Formule (II) 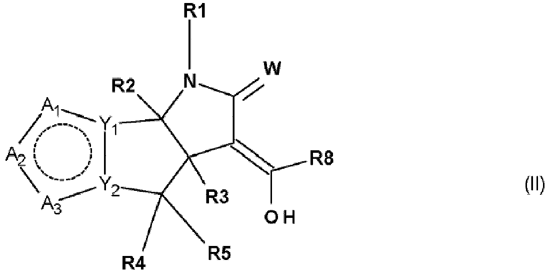 dans lequel
W est O ou S ;
R2 et R3 sont indépendamment hydrogène, ou C₁-C₃ alkyle ;
R4 et R5 sont indépendamment hydrogène, halogène, nitro, cyano, C₁-C₃ alkyle, C₁-C₃ halogénoalkyle, C₁-C₃ alcoxy, C₁-C₃ halogénoalcoxy, hydroxyle, -OC(O)R9, amine, N-C₁-C₃ alkylamine, ou N,N-di-C₁-C₃ alkylamine ;
R9 est hydrogène, C₁-C₆ alkyle, C₁-C₆ alcoxy, ou C₁-C₆ halogénoalkyle ;
R8 est hydrogène, nitro, cyano, C₁-C₆ alkyle ou C₁-C₆ halogénoalkyle ;
R1 est hydrogène, C₁-C₆ alcoxy, hydroxyle, amine, N-C₁-C₆ alkylamine, N,N-di-C₁-C₆ alkylamine, C₁-C₆, alkyle éventuellement substitué par de un à cinq R10, C₁-C₈ alkylcarbonyle ou C₁-C₈ alcoxycarbonyle, aryle éventuellement substitué par de un à cinq R10, ou hétéroaryle éventuellement substitué par de un à cinq R10, hétérocyclyle éventuellement substitué par de un à cinq R10, benzyle éventuellement substitué par de un à cinq R10, C₂-C₈- alcényle éventuellement substitué par de un à cinq R10, C₂-C₈ alcynyle éventuellement substitué par de un à cinq R10, C₃-C₇ cycloalkyle, ou C₃-C₁₀ cycloalkyle éventuellement substitué par de un à cinq R10 ;
R10 est cyano, nitro, halogène, hydroxyle, C₁-C₆ alkyle, C₁-C₆ alcoxy, C₁-C₆ halogénoalcoxy, C₁-C₆ halogénoalkyle, C₁-C₈alkylthio-, C₁-C₈halogénoalkylthio-, C₁-C₈alkylsulfinyl-, N-C₁-C₆alkylamine, N,N-di-C₁-C₆alkylamine, C₁-C₈halogénoalkylsulfinyl-, C₁-C₈alkylsulfonyl-, C₁-C₈halogénoalkylsulfonyl-, C₁-C₈alkylcarbonyl-, C₁-C₈alcoxycarbonyle, C₂-C₆alcényle ou C₂-C₆alcynyle ;
Y₁ et Y₂ sont C ;
A₁ et A₂ sont chacun indépendamment C-X, où chaque X peut être identique ou différent, à condition que A₁, A₂, A₃, Y₁ et Y₂ forment un cycle aromatique ;
A₃ est soufre ;
et X est hydrogène, halogène, cyano, nitro, hydroxyle, C₁-C₆alcoxy, C₁-C₆halogénoalcoxy, amine, N-C₁-C₆alkylamine, N,N-di-C₁-C₆alkylamine, C₁-C₆alkyle éventuellement substitué par de un à cinq R10, C₁-C₈alkylcarbonyl- ou C₁-C₈alcoxycarbonyl-, N-C₁-C₆alkylaminocarbonyle, N,N-di-C₁-C₆,alkylaminocarbonyle, aryle éventuellement substitué par de un à cinq R10, hétéroaryle éventuellement substitué par de un à cinq R10, hétérocyclyle éventuellement substitué par de un à cinq R10, C₂-C₈alcényle éventuellement substitué par de un à cinq R10, C₂-C₈alcynyle éventuellement substitué par de un à cinq R10, C₃-C₇cycloalkyle, C₃-C₁₀cycloalkyle substitué par de un à cinq R10, C₁-C₈alkylthio-, C₁-C₈halogénoalkylthio-, C₁-C₈alkylsulfinyl-, C₁-C₈halogénoalkylsulfinyl-, C₁-C₈alkylsulfonyl- ou C₁-C₈halogénoalkylsulfonyle ;
ou des sels ou des N-oxydes de celui-ci.
